# EUROPEAN PATENT SPECIFICATION

(11) **EP 3 756 543 B1**
(45) Date of publication and mention of the grant of the patent: **01.05.2024**
(21) Application number: 20178762.9
(22) Date of filing: 08.06.2020
(51) Int. Cl.: A61B 5/11, A61H 3/00, G16H 50/20

(54) **REHABILITATION SUPPORT SYSTEM, PREDICTION DEVICE, LEARNING DEVICE, METHODS, AND STORAGE MEDIUM**
REHABILITATIONSUNTERSTÜTZUNGSSYSTEM, VORHERSAGEVORRICHTUNG, LERNVORRICHTUNG, VERFAHREN UND SPEICHERMEDIUM
SYSTÈME D'AIDE À LA RÉADAPTATION, DISPOSITIF DE PRÉDICTION, DISPOSITIF D'APPRENTISSAGE, PROCÉDÉS ET SUPPORT D'ENREGISTREMENT

(30) Priority: 27.06.2019 JP 2019119947
(43) Date of publication of application: 30.12.2020
(73) Proprietor: Toyota Jidosha Kabushiki Kaisha, Toyota-shi, Aichi-ken, 471-8571 (JP)
(72) Inventor: OTSUKI, Nobuhisa, Aichi-ken, 471-8571 (JP); NAKASHIMA, Issei, Aichi-ken, 471-8571 (JP); DABA, Hiroaki, Aichi-ken, 471-8571 (JP); KOBAYASHI, Makoto, Aichi-ken, 471-8571 (JP); IMAIDA, Masayuki, Aichi-ken, 471-8571 (JP)
(74) Representative: D Young & Co LLP

(56) References cited:
- EP-A1- 3 165 208
- JP-A- 2015 159 935
- US-A1- 2014 100 494
- TAKEDA TAKAHIRO ET AL: "Evaluation of Autonomy Walk by Dynamic Foot Pressure Analysis", 2013 IEEE INTERNATIONAL CONFERENCE ON SYSTEMS, MAN, AND CYBERNETICS, IEEE, 13 October 2013 (2013-10-13), pages 3408-3413, XP032557111, DOI: 10.1109/SMC.2013.581 [retrieved on 2014-01-24]
- CHALVATZAKI GEORGIA ET AL: "LSTM-based Network for Human Gait Stability Prediction in an Intelligent Robotic Rollator", 2019 INTERNATIONAL CONFERENCE ON ROBOTICS AND AUTOMATION (ICRA), IEEE, 20 May 2019 (2019-05-20), pages 4225-4232, XP033593892, DOI: 10.1109/ICRA.2019.8793899 [retrieved on 2019-08-09]

## Description

### BACKGROUND OF THE INVENTION

### 1. Field of the Invention

The present invention relates to a rehabilitation support system, a prediction device, a learning device, methods, and a storage medium.

### 2. Description of Related Art

A technology is well-known in which a multi-joint structure for detecting a person's motion state is mounted on the person and detects a start of a fall in order to perform an action for avoiding the fall (see, for example, Japanese Unexamined Patent Application Publication No. 2010-22439).

US 2014/100494 A1 describes a Smart Gait Rehabilitation System for automated diagnosis and therapy of neurologic impairment.

EP 3 165 208 A1 describes a rehabilitation assistance device and program for controlling rehabilitation assistance device.

### SUMMARY OF THE INVENTION

When a patient (hereinafter, a trainee) performs rehabilitation, a functional independence measure (FIM) is used as an index indicating a degree of recovery of the trainee. For example, a flat ground walking FIM is used as an index indicating the degree of recovery for walking training for a patient suffering from leg paralysis. The FIM is expressed by a scale of 1 to 7 according to the amount of assistance. Further, the flat ground walking FIM indicates the walking ability of the trainee, that is, a movement ability.

The flat ground walking FIM (hereinafter, simply referred to as a walking FIM) may be assessed based on, for example, the amount of assistance when a trainee walks 50 m on flat ground (here, the walking distance may be 15 m, or the like). In other words, the walking FIM is measured by assessing the amount of assistance when the trainee walks 50 m on flat ground. However, it is impossible to frequently measure the walking FIM of a trainee with a large amount of assistance. Therefore, there is a shortcoming in that the movement ability of the trainee cannot be properly assessed.

For example, in order to measure the walking FIM, a trainee must move 50 m or more flat ground. Thus, it may be difficult for the trainee to move to a place for measuring the FIM in a hospital, or the like, where space is limited. In addition, a training staff member, such as a physiotherapist, needs to assist at all times during training. Thus, there is a shortcoming in that the index indicating the movement ability cannot be frequently measured, and thus the index cannot be properly assessed. Furthermore, since the amount of assistance by an assistant during walking on flat ground is assessed, a deviation may occur in scoring of the walking FIM depending on who the assistant (a training staff member) is.

The present disclosure provides a learning device, a prediction device, a rehabilitation support system, methods, a program, and a storage medium used for appropriately assessing a movement ability.

The invention is defined in the appended claims.

A rehabilitation support system according to a first aspect includes an actuator configured to assist a rehabilitation movement of a trainee, a sensor configured to detect data on the rehabilitation movement assisted by the actuator, and a storage medium configured to store a learned model that outputs an index indicating a movement ability of the trainee when the actuator is not used, upon receiving an input of detected data corresponding to a detection result by the sensor.

The rehabilitation support system may be a walking training system configured to perform walking training for the trainee. The actuator may assist a walking movement of the trainee. The index may be a predicted value of a walking ability of the trainee when the actuator is not used.

In the rehabilitation support system, the sensor may be provided to detect a plurality of movement amounts in the walking movement of the trainee. The rehabilitation support system may assess that the walking movement is abnormal when at least one of the movement amounts satisfies any of predetermined abnormal walking criteria. The detected data may include an assessment result on whether the walking movement is abnormal.

In the rehabilitation support system, the learned model may output the index upon receiving an input of a setting parameter on setting of the actuator.

In the rehabilitation support system, the learned model may output the index upon receiving an input of trainee data on the trainee.

In the rehabilitation support system, the actuator may assist rehabilitation movements of a plurality of trainees. The trainees may be classified into groups according to trainee data on the trainees, and a different learned model may be set for each of the groups.

A prediction device according to a second aspect predicts a movement ability of a trainee who performs rehabilitation with a rehabilitation support device. The prediction device includes a data acquisition unit configured to acquire rehabilitation data on rehabilitation of the trainee, and a prediction unit configured to predict the movement ability based on the rehabilitation data. The rehabilitation support device includes an actuator configured to assist a rehabilitation movement of the trainee, and a sensor configured to detect data on the rehabilitation movement assisted by the actuator. The rehabilitation data includes detected data corresponding to a detection result by the sensor. The prediction unit uses a learned model that outputs an index indicating the movement ability when the actuator is not used, upon receiving an input of the detected data.

A prediction method according to a third aspect predicts a movement ability of a trainee who performs rehabilitation with a rehabilitation support device. The prediction method includes a step of acquiring rehabilitation data on rehabilitation of the trainee, and a step of predicting the movement ability based on the rehabilitation data. The rehabilitation support device includes an actuator configured to assist a rehabilitation movement of the trainee, and a sensor configured to detect data on the rehabilitation movement assisted by the actuator. The rehabilitation data includes detected data corresponding to a detection result by the sensor. In the step of predicting the movement ability, a learned model is used, and the learned model outputs an index indicating the movement ability when the actuator is not used, upon receiving an input of the detected data.

A non-transitory storage medium according to a fourth aspect stores instructions that are executable by one or more processors and that cause the one or more processors to execute the prediction method.

A learning device according to a fifth aspect the includes a data acquisition unit configured to acquire rehabilitation data from a rehabilitation support device having an actuator configured to assist a rehabilitation movement of a trainee and a sensor configured to detect data on the rehabilitation movement assisted by the actuator, a data generation unit configured to generate, as data for learning, an index indicating a movement ability of the trainee and the rehabilitation data that includes detected data corresponding to a detection result by the sensor, and a learning unit configured to generate, by performing machine learning using the data for learning, a learning model that outputs an index indicating a movement ability of the trainee when the actuator is not used, upon receiving an input of the detected data.

In the learning device, the rehabilitation support device may be a walking training device configured to perform walking training for the trainee. The actuator may assist a walking movement of the trainee. The rehabilitation data may include, as an index indicating the movement ability of the trainee, an actual measurement value of a walking ability of the trainee when the actuator is not used. The learning unit may perform learning using the actual measurement value of the walking ability as supervised data.

In the learning device, the sensor may be provided to detect a plurality of movement amounts in the walking movement of the trainee. The rehabilitation support device may assess that the walking movement is abnormal when at least one of the movement amounts satisfies any of predetermined abnormal walking criteria. The rehabilitation data may include an assessment result on whether the walking movement is abnormal as the detected data.

In the learning device, the data acquisition unit may acquire a setting parameter on setting of the actuator as the rehabilitation data. The learning unit may generate a learning model that outputs the index upon receiving an input of the setting parameter.

In the learning device, the learning unit may generate a learning model that outputs the index upon receiving an input of trainee data on the trainee.

In the learning device, the actuator may assist rehabilitation movements of a plurality of trainees. The trainees may be classified into groups according to trainee data on the trainees, and the learning unit may generate a different learning model for each of the groups.

A storage medium according to a sixth aspect stores a learned model that is executable by one or more processors and that causes the one or more processors to function so as to predict a movement ability of a trainee based on rehabilitation data for assessment acquired by a rehabilitation support device. The learned model is a learning model generated by the learning device.

In the learned model, the detected data and the index may be learned in association with each other.

A learning method according to a seventh aspect includes a step of acquiring rehabilitation data from a rehabilitation support device having an actuator configured to assist a rehabilitation movement of a trainee and a sensor configured to detect data on the rehabilitation movement assisted by the actuator, a step of generating, as data for learning, an index indicating a movement ability of the trainee and the rehabilitation data that includes detected data corresponding to a detection result by the sensor, and a step of generating, by performing machine learning using the data for learning, a learning model that outputs an index indicating a movement ability of the trainee when the actuator is not used, upon receiving an input of the detected data.

A storage medium according to an eighth aspect stores instructions that are executable by one or more processors and that cause the one or more processors to execute the learning method.

An operation method according to a ninth aspect is for operating a rehabilitation support system including an actuator configured to assist a rehabilitation movement of a trainee and a sensor configured to detect data on the rehabilitation movement assisted by the actuator. The operation method includes a step of acquiring detected data corresponding to a detection result by the sensor and a step of outputting an index indicating a movement ability of the trainee when the actuator is not used, upon receiving an input of the detected data.

With each aspect of the present disclosure it is possible to provide a rehabilitation support system, a prediction device, a learning device, methods, and a storage medium used for appropriately assessing a movement ability.

### BRIEF DESCRIPTION OF THE DRAWINGS

Features, advantages, and technical and industrial significance of exemplary embodiments will be described below with reference to the accompanying drawings, in which like signs denote like elements, and wherein:
FIG. 1 is a schematic perspective view of a walking training device according to the present embodiment;
FIG. 2 is a schematic perspective view illustrating a walking assistive device;
FIG. 3 is a diagram illustrating a system configuration of the walking training device;
FIG. 4 is a block diagram illustrating a configuration of a server;
FIG. 5 is a flowchart for describing processing in a control unit;
FIG. 6 is a table describing a data set for learning;
FIG. 7 is a diagram for describing a learning model;
FIG. 8 is a flowchart illustrating processing according to a second embodiment;
FIG. 9 is a block diagram illustrating a configuration of the server according to a third embodiment;
FIG. 10 is a graph illustrating statistical values of indices indicating a degree of recovery;
FIG. 11 is a flowchart illustrating processing according to the third embodiment;
FIG. 12 is a diagram describing a first abnormal walking criterion;
FIG. 13 is a diagram describing a second abnormal walking criterion;
FIG. 14 is a diagram describing a third abnormal walking criterion;
FIG. 15 is a diagram describing a fourth abnormal walking criterion;
FIG. 16 is a diagram describing a fifth abnormal walking criterion;
FIG. 17 is a diagram describing a sixth abnormal walking criterion;
FIG. 18 is a diagram describing a seventh abnormal walking criterion;
FIG. 19 is a flow diagram illustrating processing operation of the walking training device;
FIG. 20 is a table illustrating determination results of abnormal walking;
FIG. 21 is a table illustrating changes in setting parameters;
FIG. 22 is a diagram for describing a learning model using a recurrent neural network (RNN);
FIG. 23 is a diagram for describing a detected data used in an embodiment example; and
FIG. 24 is a diagram for describing a learning model used in a second embodiment example.

### DETAILED DESCRIPTION OF EMBODIMENTS

Hereinafter, the present invention will be described using exemplary illustrative embodiments, where the invention is however only limited by the claims. In addition, all of the configurations described in the embodiments are not necessarily essential for solving problems.

### First Embodiment

Hereinafter, a first embodiment will be described with reference to the drawings.

### System Configuration

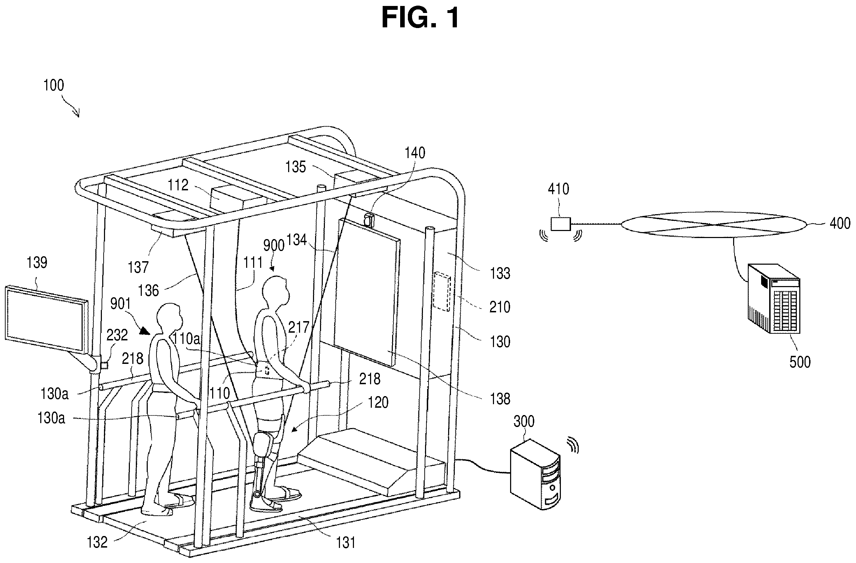

FIG. 1 is an overall conceptual diagram illustrating a configuration example of a rehabilitation support system according to a first embodiment. The rehabilitation support system (a rehabilitation system) according to the present embodiment is mainly composed of a walking training device 100, an external communication device 300, and a server (a server device) 500. The server 500 may be an external server that can communicate with the walking training device 100 or may be imbedded in a main body of the walking training device 100. In other words, the walking training device 100 may or may not include the server 500. A learned model to be described below may be imbedded in the external server 500 or the walking training device 100.

The walking training device 100 is a specific example of a rehabilitation support device that supports rehabilitation of a trainee (user) 900. The walking training device 100 is a device for a trainee 900, who is a hemiplegic patient suffering from paralysis of one leg, to perform walking training following guidance of a training staff member 901. Here, the training staff member 901 may be a therapist (a physiotherapist) or a doctor, and may also be referred to as, for example, a training guide, a training assistant, or a training helper since he/she guides or assists the training of the trainee.

The walking training device 100 mainly includes a control panel 133 attached to a frame 130 that forms an entire structure, a treadmill 131 on which the trainee 900 walks, and a walking assistive device 120 to be worn on an injured leg that is a paralyzed leg of the trainee 900.

The frame 130 is erected on a treadmill 131 installed on the ground. The treadmill 131 rotates a ring-shaped belt 132 by a motor (not shown). The treadmill 131 is a device that prompts the trainee 900 to walk. The trainee 900 who performs walking training gets onto the belt 132 and attempts a walking movement according to a movement of the belt 132. For example, although the training staff member 901 can perform the walking movement together on the belt 132 behind the trainee 900 as illustrated in FIG. 1, the training staff member 901 may usually be in a state where it is easy to assist the trainee 900, such as a state where the training staff member 901 stands with one foot on each side of the belt 132.

The frame 130 supports, for example, a control panel 133 that houses a total control unit 210 that controls a motor or a sensor, a training monitor 138 that may be, for example, a liquid crystal panel, which presents progress of training, and the like, to the trainee 900. Further, the frame 130 supports a front pulling unit 135 near the front of the top of the head of the trainee 900, a harness pulling unit 112 near the top of the head, and a rear pulling unit 137 near the rear of the top of the head, respectively. Moreover, the frame 130 includes handrails 130a for the trainee 900 to hold.

The handrails 130a are arranged on the right and left sides of the trainee 900. Each of the handrails 130a is arranged in a direction parallel to the walking direction of the trainee 900. The handrail 130a may be adjusted vertically and horizontally. In other words, the handrail 130a may include a mechanism that changes the height and width thereof. Furthermore, the handrail 130a may be composed such that its inclination angle is changed by adjusting, for example, the heights of the front side and the rear side in the walking direction to be different. For example, the handrail 130a can have an inclination angle that becomes gradually higher along the walking direction.

In addition, the handrail 130a is provided with a handrail sensor 218 that detects a load received from the trainee 900. For example, the handrail sensor 218 may be a load detection sheet of a resistance change detection type in which electrodes are arranged in a matrix. Alternatively, the handrail sensor 218 may be a six-axis sensor in which a three axis acceleration sensor (x, y, z) and three axis gyro sensors (roll, pitch, yaw) are combined. However, the type and an installed position of the handrail sensor 218 are not limited.

A camera 140 functions as an image capturing unit for observing the whole body of the trainee 900. The camera 140 is provided near the training monitor 138 so as to face the trainee. The camera 140 captures a still image or a moving image of the trainee 900 performing training. The camera 140 includes a set of an image capturing element and a lens that has a viewing angle enough to capture the whole body of the trainee 900. The image capturing element may be, for example, a complementary metal-oxide-semiconductor (CMOS) image sensor, and converts an optical image formed on an imaging surface into an image signal.

By a cooperative operation between the front pulling unit 135 and the rear pulling unit 137, a load of the walking assistive device 120 is offset such that the load does not become a burden on the injured leg, and further, a swing movement of the injured leg is assisted according to the degree of setting.

One end of a front wire 134 is connected to a winding mechanism of the front pulling unit 135 and the other end thereof is connected to the walking assistive device 120. The winding mechanism of the front pulling unit 135 winds or unwinds the front wire 134 according to the movement of the injured leg by turning on/off a motor (not shown). Similarly, one end of a rear wire 136 is connected to a winding mechanism of the rear pulling unit 137 and the other end thereof is connected to the walking assistive device 120. The winding mechanism of the rear pulling unit 137 winds or unwinds the rear wire 136 according to the movement of the injured leg by turning on/off a motor (not shown). By the cooperative operation between the front pulling unit 135 and the rear pulling unit 137, the load of the walking assistive device 120 is offset such that the load does not become a burden on the injured leg, and further, the swing movement of the injured leg is assisted according to the degree of setting.

For example, as an operator, the training staff member 901 sets a high assisting level for a trainee with severe paralysis. When the assisting level is set high, the front pulling unit 135 winds up the front wire 134 with a relatively large force according to the swing timing of the injured leg. When training progresses and assistance is no longer needed, the training staff member 901 sets the assisting level to the minimum. When the assisting level is set to the minimum, the front pulling unit 135 winds the front wire 134 with a force enough to cancel the weight of the walking assistive device 120 according to the swing timing of the injured leg.

The walking training device 100 includes a fall prevention harness device as a safety device, which is mainly composed of a brace 110, a harness wire 111, and a harness pulling unit 112. The brace 110 is a belt wound around the abdomen of the trainee 900 and is fixed to the waist by, for example, a hook-and-loop fastener. The brace 110 includes a connection hook 110a to which one end of a harness wire 111 is connected as a hanging tool, and may be referred to as a hanger belt. The trainee 900 wears the brace 110 such that the connection hook 110a is positioned on the back.

One end of the harness wire 111 is connected to the connection hook 110a of the brace 110, and the other end thereof is connected to the winding mechanism of the harness pulling unit 112. The winding mechanism of the harness pulling unit 112 winds or unwinds the harness wire 111 by turning on/off a motor (not shown). With such a configuration, when the trainee 900 is about to fall, the fall prevention harness device prevents the trainee 900 from falling by winding up the harness wire 111 according to an instruction of the total control unit 210 that has detected the movement and supporting the upper body of the trainee 900 with the brace 110.

The brace 110 includes a posture sensor 217 that detects a posture of the trainee 900. The posture sensor 217 may be, for example, a combination of the gyro sensor and the acceleration sensor, and outputs an inclination angle of the abdomen around which the brace 110 is wound with respect to the direction of gravity.

A management monitor 139 is attached to the frame 130 and is a display input device that is mainly used by the training staff member 901 for monitoring and operating. The management monitor 139 may be, for example, a liquid crystal panel, and a touch panel is provided on a surface thereof. The management monitor 139 displays various menu items on a training setting, various parameter values during training, training results, and the like. Moreover, an emergency stop button 232 is provided near the management monitor 139. The walking training device 100 performs an emergency stop when the training staff member 901 presses the emergency stop button 232.

The walking assistive device 120 is worn on the injured leg of the trainee 900 and assists walking of the trainee 900 by reducing a load on a knee joint of the injured leg due to extension and flexion. The walking assistive device 120 includes a sensor that measures a load on a sole, and the like, and outputs various pieces of data on moving a leg to the total control unit 210. In addition, the brace 110 can be connected to the walking assistive device 120, using a connection member (hereinafter, a hip joint) having a rotation unit. Details of the walking assistive device 120 will be described below.

The total control unit 210 generates rehabilitation data that may include setting parameters on the training setting, various pieces of data on the moving leg which are output as the training results from the walking assistive device 120, and the like. The rehabilitation data may include data indicating the training staff member 901, his/her years of experience or a level of skill, or the like, data indicating a symptom, a walking ability, a recovery degree, and the like, of the trainee 900, and various pieces of data output from a sensor provided outside the walking assistive device 120, and the like. Details of the rehabilitation data will be described below.

The external communication device 300 is a specific example of a transmitter that transmits the rehabilitation data to the outside. The external communication device 300 may have a function of receiving and temporarily storing the rehabilitation data output from the walking training device 100, and a function of transmitting the stored rehabilitation data to the server 500.

The external communication device 300 is connected to the control panel 133 of the walking training device 100 via, for example, a universal serial bus (USB) cable. Moreover, the external communication device 300 is connected to a network 400, such as the Internet or an intranet, via a wireless communication device 410, for example, in the form of a wireless local area network (LAN). In addition, the walking training device 100 may include a communication device instead of the external communication device 300.

The server 500 is a specific example of a storage medium that stores the rehabilitation data. The server 500 is connected to the network 400 and has a function of accumulating the rehabilitation data received from the external communication device 300. Functions of the server 500 will be described below.

In the first embodiment, although the walking training device 100 was described as an example of the rehabilitation support device, the rehabilitation support device is not limited thereto, and may be any rehabilitation support device that supports rehabilitation of the trainee. For example, the rehabilitation support device may be an upper extremity rehabilitation support device that supports rehabilitation of a shoulder or an arm. Alternatively, the rehabilitation support device may be a rehabilitation support device that supports rehabilitation of a balance ability of the trainee.

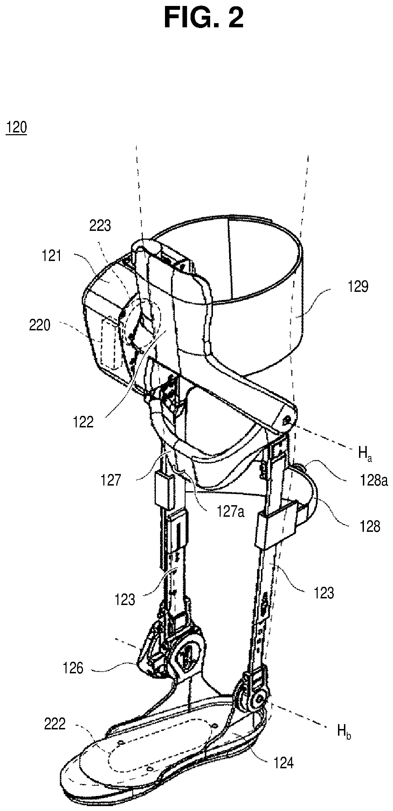

Next, the walking assistive device 120 will be described with reference to FIG. 2. FIG. 2 is a schematic perspective view illustrating a configuration example of the walking assistive device 120. The walking assistive device 120 mainly includes a control unit 121, a plurality of frames supporting each part of the injured leg, and a load sensor 222 detecting a load on a sole.

The control unit 121 includes an auxiliary control unit 220 that controls the walking assistive device 120, and a motor (not shown) that generates a driving force for assisting the extension and flexion movements of the knee joint. The frames supporting each part of the injured leg include an upper leg frame 122, and a lower leg frame 123 rotatably connected to the upper leg frame 122. Moreover, the frames include a sole frame 124 rotatably connected to the lower leg frame 123, a front connection frame 127 to be connected to the front wire 134, and a rear connection frame 128 to be connected to the rear wire 136.

The upper leg frame 122 and the lower leg frame 123 relatively rotate around a hinge axis Hₐ, illustrated in FIG. 2. A motor of the control unit 121 rotates according to an instruction of the auxiliary control unit 220, thereby applying a force to the upper leg frame 122 and the lower leg frame 123, such that relatively open or close around the hinge axis Hₐ. An angle sensor 223 housed in the control unit 121 may be, for example, a rotary encoder, and detects an angle formed by the upper leg frame 122 and the lower leg frame 123 around the hinge axis Hₐ. The lower leg frame 123 and the sole frame 124 relatively rotate around the illustrated hinge axis H_{b}. The angle range of relative rotation is adjusted in advance by an adjustment mechanism 126.

The front connection frame 127 is provided so as to extend in a horizontal direction on the front of the upper leg, and to be connected to the upper leg frame 122 at both ends. In addition, the front connection frame 127 is provided with a connection hook 127a to be connected to the front wire 134 near the center in the horizontal direction thereof. The rear connection frame 128 is provided so as to extend in the horizontal direction on the rear of the lower leg, and to be connected to the lower leg frame 123 extending vertically at both ends. Moreover, the rear connection frame 128 is provided with a connection hook 128a to be connected to the rear wire 136 near the center in the horizontal direction thereof.

The upper leg frame 122 includes an upper leg belt 129. The upper leg belt 129 is provided integrally with the upper leg frame, and is wound around the upper leg of the injured leg to fix the upper leg frame 122 to the upper leg. As such, the entire walking assistive device 120 is prevented from being displaced from the leg of the trainee 900.

The load sensor 222 is embedded in the sole frame 124. The load sensor 222 may be configured to calculate, for example, a center of pressure (COP) by detecting the magnitude and distribution of a vertical load on the sole of the trainee 900. The load sensor 222 may be, for example, the load detection sheet of the resistance change detection type in which electrodes are arranged in a matrix.

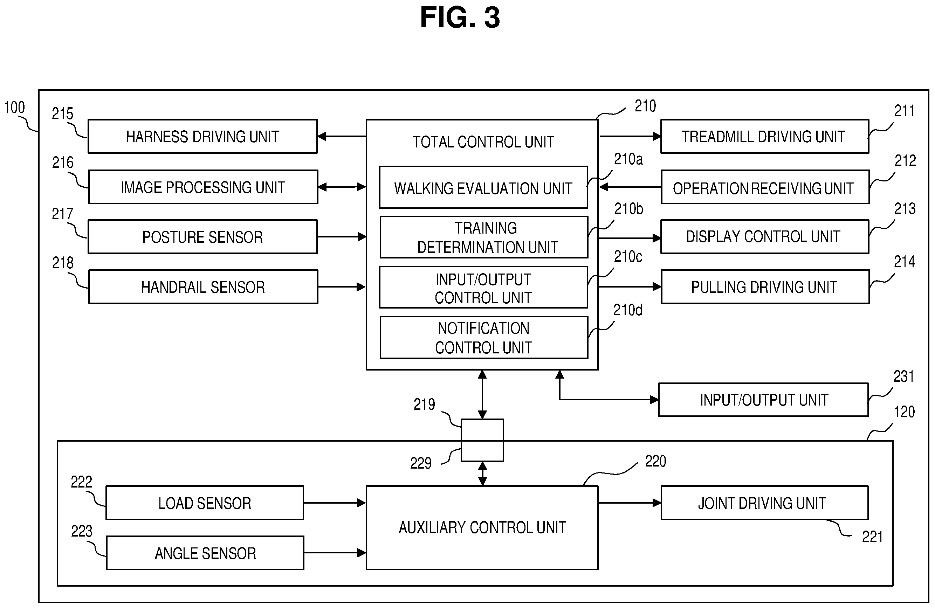

Next, an example of a system configuration of the walking training device 100 will be described with reference to FIG. 3. FIG. 3 is a block diagram illustrating an example of the system configuration of the walking training device 100. As illustrated in FIG. 3, the walking training device 100 may include the total control unit 210, a treadmill driving unit 211, an operation receiving unit 212, a display control unit 213, and a pulling driving unit 214. The walking training device 100 may further include a harness driving unit 215, an image processing unit 216, the posture sensor 217, the handrail sensor 218, a communication connection interface (IF) 219, an input/output unit 231, and a walking assistive device 120.

The total control unit 210 may be, for example, a micro-processing unit (MPU), and controls the entire device by performing a control program read from a system memory. The total control unit 210 may include a walking assessment unit 210a, a training determination unit 210b, an input/output control unit 210c, and a notification control unit 210d to be described below.

The treadmill driving unit 211 includes a motor that rotates the belt 132 and a driving circuit thereof. The total control unit 210 performs rotation control of the belt 132 by sending a driving signal to the treadmill driving unit 211. The total control unit 210 may adjust the rotation speed of the belt 132, for example, according to walking speed set by the training staff member 901.

The operation receiving unit 212 receives an input operation by the training staff member 901 and transmits an operation signal to the total control unit 210. The training staff member 901 operates an operation button provided in the device, a touch panel superpositioned on the management monitor 139, an attached remote controller, and the like, which compose the operation receiving unit 212. With the above operation, it is possible to give an instruction to turn on/off power or start training, to input numerical values on settings, or to select menu items. Alternatively, the operation receiving unit 212 may also receive an input operation by the trainee 900.

The display control unit 213 receives a display signal from the total control unit 210, generates a display image, and displays the display image on the training monitor 138 or the management monitor 139. The display control unit 213 generates an image showing the progress of the training or a real-time video captured by the camera 140 according to the display signal.

The pulling driving unit 214 includes a motor used for pulling the front wire 134 and a driving circuit thereof, which compose the front pulling unit 135, and a motor used for pulling the rear wire 136 and a driving circuit thereof, which compose the rear pulling unit 137. The total control unit 210 controls each of winding of the front wire 134 and winding of the rear wire 136 by sending a driving signal to the pulling driving unit 214. In addition, the total control unit 210 controls a pulling force of each wire by controlling driving torque of the motors in addition to the winding, and may be performed to. The total control unit 210 assists a swinging movement of the injured leg by, for example, identifying the timing at which the injured leg switches from a stance state to a swing state based on the detection result by the load sensor 222, and increasing/decreasing the pulling force of each wire in synchronization with the timing.

The harness driving unit 215 includes a motor used for pulling the harness wire 111 and a driving circuit thereof, which compose the harness pulling unit 112. The total control unit 210 controls the winding and the pulling force of the harness wire 111 by sending a driving signal to the harness driving unit 215. For example, when predicting a fall of the trainee 900, the total control unit 210 winds up the harness wire 111 by a certain amount, thereby preventing the trainee from falling.

The image processing unit 216 is connected to the camera 140 and can receive an image signal from the camera 140. According to an instruction of the total control unit 210, the image processing unit 216 receives an image signal from the camera 140 and generates image data performing image processing on the received image signal. Further, the image processing unit 216 can also perform a specific image analysis by performing the image processing on an image signal received from the camera 140 according to an instruction of the total control unit 210. For example, the image processing unit 216 detects a position (a standing position) of the foot of the injured leg in contact with the treadmill 131 using an image analysis. Specifically, for example, the image processing unit 216 calculates the stance position by extracting an image area near the tip of the sole frame 124 and analyzing an identification marker drawn on the belt 132 overlapping the tip.

As described above, the posture sensor 217 detects the inclination angle of the abdomen of the trainee 900 with respect to the direction of gravity, and transmits a detection signal to the total control unit 210. The total control unit 210 calculates the posture of the trainee 900, specifically, the inclination angle of the trunk of the trainee 900, using the detection signal from the posture sensor 217. Moreover, the total control unit 210 may be connected to the posture sensor 217 in a wired manner or via a near-field wireless communication.

The handrail sensor 218 detects the load on the handrail 130a. In other words, a load corresponding to the weight of the trainee 900 that cannot be supported with both of his/her own legs is applied to the handrail 130a. The handrail sensor 218 detects the load on the handrail 130a and transmits a detection signal to the total control unit 210.

The total control unit 210 also has a function as an execution unit that executes various calculations, or the like related to control. The walking assessment unit 210a assesses whether the walking movement of the trainee 900 is abnormal, using data acquired from various sensors. The training determination unit 210b determines a training result of a series of walking training based on, for example, the integrated number of abnormal walking assessed by the walking assessment unit 210a. The total control unit 210 can generate the determination result, the integrated number of abnormal walking which is a source of the determination result, or the like, as part of the rehabilitation data.

Further, a method for a determination, including criteria for the above determination, is not limited. For example, a determination may be made by comparing a movement amount of a paralyzed part with a criterion thereof for each walking phase. The walking phase refers to, for example, a stance phase in a stance state, a transition phase from the stance phase to a swing phase in a swing state, the swing phase, and a transition phase from the swing phase to the stance phase, which are classified from one walking cycle of the injured leg (or a healthy leg). It is possible to classify which walking phase is based on the detection result by, for example, the load sensor 222 as described above. Moreover, although for the walking cycle, the stance phase, the transition phase, the swing phase, and the transition phase can be handled as one walking cycle as described above, it does not matter which phase is defined as a start phase. In addition, for example, for the walking cycle, a state of supporting both legs, a state of supporting a single leg (the injured leg), the state of supporting both legs, and the state of supporting a single leg (the healthy leg) can be handled as one walking cycle. In this case, it does not matter which walking phase is defined as a start phase, either.

Moreover, the walking cycle focusing on the right leg or the left leg (the healthy leg or the injured leg) may be further subdivided. For example, the stance phase can be expressed by being divided into an initial contact and four phases, and the swing phase can be expressed by being divided into three phases. The initial contact refers to a moment when an observed foot contacts the ground, and the four stance phases refer to a loading response phase, a mid-stance phase, a terminal stance phase, and a pre-swing phase. The loading response phase is a period from the initial contact to a moment when the opposite foot lifts off the ground (opposite foot off the ground). The mid-stance phase is a period from the moment when the opposite foot lifts off the ground to a moment when the heel of the observed foot lifts off the ground (heel off the ground). The terminal stance phase is a period from the moment when the heel lifts off the ground to the initial contact of the opposite foot. The pre-swing period is a period from the initial contact of the opposite foot to a moment when the observed foot lifts off the ground (off the ground). The three swing phases refer to an initial swing phase, a mid-swing phase, and a terminal swing phase. The initial swing phase is a period from the end of the pre-swing phase (the off-the-ground) to a moment when both feet cross (feet crossing). The mid-swing phase is a period from the feet crossing to a moment when the tibia is vertical to the ground (vertical tibia). The terminal swing is a period from the vertical tibia to the next initial contact.

The communication connection IF 219 is connected to the total control unit 210 to give a command to the walking assistive device 120 worn on the injured leg of the trainee 900 or receive sensor information.

The walking assistive device 120 can include a communication connection IF 229 connected to the communication connection IF 219 in a wired or wireless manner. The communication connection IF 229 is connected to the auxiliary control unit 220 of the walking assistive device 120. The communication connection IFs 219, 229 are communication interfaces, such as a wired LAN and a wireless LAN, according to a communication standard.

Further, the walking assistive device 120 may include the auxiliary control unit 220, a joint driving unit 221, the load sensor 222, and the angle sensor 223. The auxiliary control unit 220 may be, for example, an MPU, and controls the walking assistive device 120 by performing a control program received from the total control unit 210. Moreover, the auxiliary control unit 220 notifies the total control unit 210 of the state of the walking assistive device 120 via the communication connection IFs 219, 229. In addition, the auxiliary control unit 220 receives a command from the total control unit 210 and controls starting, stopping, or the like, of the walking assistive device 120.

The joint driving unit 221 includes a motor of the control unit 121 and a driving circuit thereof. The auxiliary control unit 220 sends a driving signal to the joint driving unit 221, thereby applying a force to the upper leg frame 122 and the lower leg frame 123 such that they relatively open or close around the hinge axis Hₐ. With such an operation, the extension and the flexion movements of the knee are assisted, and the knee is prevented from giving way.

The load sensor 222 detects the magnitude and distribution of the vertical load on the sole of the trainee 900 as described above, and transmits a detection signal to the auxiliary control unit 220. The auxiliary control unit 220 determines whether the state of the leg is the swinging state or the standing state, predicts switching the state of the legs, or the like, by receiving and analyzing the detection signal.

The angle sensor 223 detects the angle formed by the upper leg frame 122 and the lower leg frame 123 around the hinge axis Hₐ as described above, and transmits a detection signal to the auxiliary control unit 220. The auxiliary control unit 220 receives the detection signal and calculates an open angle of the knee joint.

The input/output unit 231 may include, for example, a USB interface, and is a communication interface for connecting to an external device (the external communication device 300 or other external devices). The input/output control unit 210c of the total control unit 210 communicates with the external device via the input/output unit 231 to rewrite a control program in the total control unit 210 described above and a control program in the auxiliary control unit 220, receive a command, output generated rehabilitation data, and the like. The walking training device 100 communicates with the server 500 via the input/output unit 231 and the external communication device 300 under the control of the input/output control unit 210c. For example, the input/output control unit 210c can control transmission of the rehabilitation data to the server 500 or reception of a command from the server 500 via the input/output unit 231 and the external communication device 300.

The notification control unit 210d performs notification through the management monitor 139 or a separately provided speaker by controlling the display control unit 213, a separately provided voice control unit, and the like, when notification to the training staff member 901 is needed. Although details of the notification will be described below, the time when the notification to the training staff member 901 is needed may be a time when a command for performing the notification is received from the server 500.

Next, details of the server 500 will be described. As described above, the walking training device 100 transmits various pieces of rehabilitation data to the server 500 via the external communication device 300. The server 500 may be configured to receive the rehabilitation data from a plurality of walking training devices 100, and thereby can collect a large amount of rehabilitation data. The server 500 is a processing device that processes various pieces of data. For example, the server 500 may function as a learning device (a learning tool) that establishes a learned model by performing machine learning using the collected rehabilitation data. Moreover, the learning device may also be referred to as a learning model generation device.

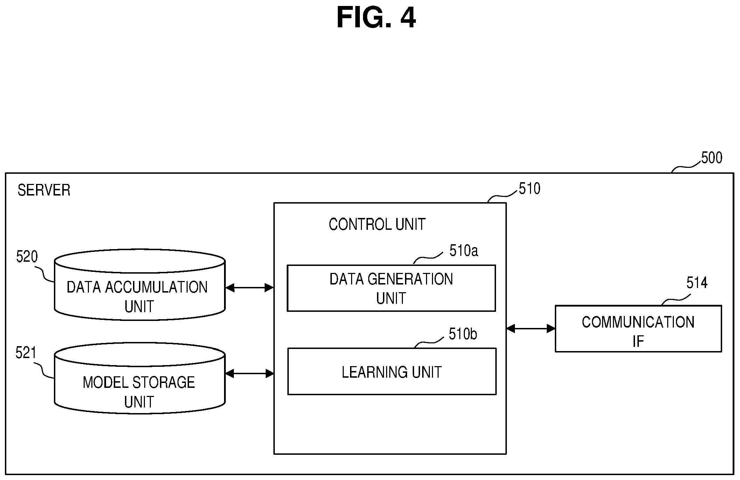

FIG. 4 is a block diagram illustrating a configuration example of the server 500. As illustrated in FIG. 4, the server 500 may include a control unit 510, a communication IF 514, a data accumulation unit 520, and a model storage unit 521. The control unit 510 may be, for example, an MPU, and controls the server 500 by performing a control program read from a system memory. The control unit 510 includes a data generation unit 510a and a learning unit 510b. In this case, the control program includes a program for realizing the functions of the data generation unit 510a and the learning unit 510b.

The communication IF 514 may include, for example, a wired LAN interface and is a communication interface for connecting to the network 400. The control unit 510 can receive the rehabilitation data from the walking training device 100 via the communication IF 514 and transmit a command to the walking training device 100.

The data accumulation unit 520 has a storage device, such as a hard disk drive (HDD) and thereby can collect a large amount of rehabilitation data a solid state drive (SSD), and stores the rehabilitation data. The control unit 510 writes, in the data accumulation unit 520, the rehabilitation data received from the external communication device 300 via communication IF 514. The communication IF 514 and the data accumulation unit 520 function as, for example, a data acquisition unit that acquires the rehabilitation data.

The model storage unit 521 also has a storage device, such as an HDD and an SSD. In addition, the data accumulation unit 520 and the model storage unit 521 may have a common storage device. The model storage unit 521 stores at least one of an unlearned learning model (including a model in which learning is being performed) (hereinafter, an unlearned model) and a learned learning model (hereinafter, a learned model). When the server 500 functions as a learning device, at least the unlearned model is stored in the model storage unit 521. When the server 500 performs a rehabilitation support process in cooperation with the walking training device 100, at least an operable learned model is stored in the model storage unit 521.

Further, the control unit 510 can be configured to control switching between a function as a learning device and a function of performing the rehabilitation support process using the learned model. However, the server 500 can be distributed to a device used in a learning stage and a device used in an operation stage in which the learned model is used. The data generation unit 510a and the learning unit 510b are provided to cause the server 500 to function as a learning device.

### Rehabilitation Data

Here, before describing the data generation unit 510a and the learning unit 510b, the rehabilitation data that can be collected by the server 500 for learning or for the rehabilitation support process will be described. The rehabilitation data that can be collected by the server 500 mainly includes (1) setting parameters of the walking training device 100, (2) data detected by a sensor, and the like, provided in the walking training device 100, (3) data on the trainee 900, and (4) data on the training staff member 901. The rehabilitation data of (1) to (4) may be collected in association with an acquired date and time. Further, the detected data or the setting parameters may be collected as time-series log data. Alternatively, the rehabilitation data may be a feature amount, or the like, extracted from data at predetermined time intervals.

The rehabilitation data is acquired mainly by an operation input, an automatic input, measurement by a sensor, and the like, in the walking training device 100. Moreover, the rehabilitation data may include recorded data recorded by the camera 140. Further, the rehabilitation data may be data of each date when rehabilitation is performed, in which case, the rehabilitation data may be referred to as daily report data. Hereinbelow, description will be given on the assumption that the server 500 collects the rehabilitation data generated in the walking training device 100. However, the server 500 can also be configured to acquire part of the rehabilitation data from, for example, a server other than the walking training device 100. Here, the part of the rehabilitation data may be, for example, details of the data of the above (3), such as a symptom of the trainee 900, or details of the data of the above (4), such as years of experience of a PT. The former can be stored in another server as medical record information of the trainee 900, and the latter can be stored in yet another server as a resume of the trainer, or the like.

In the learning stage, the server 500 receives the rehabilitation data from the walking training device 100 at the time of generation of the rehabilitation data, or periodically, for example, every day or every week. The type of rehabilitation data (content contained in the rehabilitation data) to be used in the learning stage may be different from that of the rehabilitation data to be used in the operation stage. For example, in the operation stage, the server 500 receives the rehabilitation data from the walking training device 100 at the start of training, and receives data which has been changed during training among the above (1) and (2). Further, the transmission and reception of the rehabilitation data may be performed mainly by any one of the walking training device 100 or the server 500.

The above (1) will be described. The data of the above (1) may be defined as the training data of the trainee 900 acquired in the walking training device 100 during execution of the rehabilitation, together with the detected data of the above (2).

The setting parameters of the walking training device 100 may be, for example, data input by an operator or automatically set data for setting movements of the walking training device 100. In addition, as described above, the operator is usually the training staff member 901 who actually attends on the training of the trainee 900, and description below will be made on the assumption that the operator is the training staff member 901. In addition, since the training staff member 901 is often the physical therapist (PT), the training staff member 901 may be simply referred to as a "PT" below.

The setting parameters relate to setting of an actuator. In the walking training device 100, the difficulty of the walking training can be adjusted according to the setting parameters. For example, the total control unit 210 controls an actuator, such as a motor, according to the setting parameters. Moreover, the setting parameters may include a parameter indicating the level of difficulty, in which case, some or all of the other setting parameters can be changed accompanied with the change in the level. The training staff member 901 increases the difficulty of walking training as the recovery of the trainee 900 progresses. In other words, the training staff member 901 reduces the assistance by the walking training device 100 as the walking ability of the trainee 900 increases. On the contrary, the training staff member 901 may increase the assistance when an abnormality is found during walking training. By appropriate adjustment of the setting parameters by the training staff member 901, the trainee 900 can perform appropriate walking training and thus perform rehabilitation more efficiently.

Specific examples of the setting parameters are described below. Examples of the setting parameters may include a partial weight load support amount [%], vertical and horizontal positions [cm] of the handrail 130a, presence/absence of the hip joint, a plantarflexion limitation [deg], a dorsiflexion limitation [deg], treadmill speed [km/h], swing assistance [level], and a front-to-rear ratio during swing [front/rear], knee extension assistance [level], a knee flexion angle [deg], knee flexion/extension time [sec], complement height [mm], an unload threshold [%], and a load threshold [%]. Moreover, the units of the data included in the rehabilitation data, including the setting parameters exemplified here, does not matter.

The partial weight load support amount is a rate at which the weight of the trainee 900 is released when the harness pulling unit 112 pulls the harness wire 111. The training staff member 901 sets the partial weight load support amount to a lower value as the desired difficulty of the walking training becomes higher. The vertical and horizontal positions of the handrail 130a are amounts adjusted from a reference position of the handrail 130a. The presence/absence of the hip joint depends on whether the hip joint is attached. The plantarflexion and the dorsiflexion limitations define angle ranges within which the lower leg frame 123 and the sole frame 124 can rotate around the hinge axis H_{b}. The plantarflexion limitation corresponds to the upper limit angle of the front of the ankle and the dorsiflexion limitation corresponds to the maximum angle of the rear of the ankle. In other words, the plantarflexion and the dorsiflexion limitations are respectively limit values of angles at which the ankle is bent toward the direction where the toe is lowered and the direction where the toe is raised. The training staff member 901 sets the values of the plantarflexion and the dorsiflexion limitations, such that the angle ranges become larger as the difficulty of the desired walking training becomes higher.

The treadmill speed is a walking speed of the treadmill 131. The training staff member 901 sets the treadmill speed to a higher value as the difficulty level of the desired walking training becomes higher. The swing assistance is a level corresponding to the pulling force applied by the front wire 134 when the leg swings. The higher the level becomes, the larger the maximum pulling force becomes. The training staff member 901 sets the swing assistance to a lower level as the difficulty of the desired walking training becomes higher. The front-to-rear ratio during swing is the ratio of the pulling force of the front wire 134 to the pulling force of the rear wire 136, when the leg swings.

The knee extension assistance is a level corresponding to the driving torque of the joint driving unit 221 applied to prevent the knee from giving way during standing. The driving torque increases as the level increases. The training staff member 901 sets the knee extension assistance to a lower level as the difficulty of the desired walking training becomes higher. The knee flexion angle is an angle of the knee when performing knee extension assistance. The knee flexion/extension time is a period during which the knee extension assistance is performed. When the value thereof is large, the knee is assisted to flex/extend the knee slowly, and when the value thereof is small, the knee is assisted to flex/extend quickly.

The height addition is the height of a member, such as a cushion, provided on the sole of a shoe of the opposite leg (the leg to which the walking assistive device 120 is not attached) to the paralyzed leg of the trainee 900. The unload threshold is one of the thresholds of the load applied to the sole, and when a load falls below the threshold, the swing assistance is released. The load threshold is one of the thresholds of the load applied to the sole, and when a load exceeds the threshold, the swing assistance is performed. As such, the walking assistive device 120 can be configured to adjust flexion/extension movements of the knee using the four setting parameters, that is, the knee flexion angle, the knee flexion/extension time, the unload threshold, and the load threshold.

In addition, the walking training device 100 may be configured to give feedback on set values and target values of various parameters, such as a load and an angle, a target achievement rate, and a target achievement timing via, for example, audio through a speaker (not shown). The above parameters may also include parameters for setting, for example, the presence/absence of the feedback sound and the volume.

Moreover, the above setting parameters do not have to be directly related to the training. For example, the above setting parameters may be a set value of an image, music, game types, game difficulty levels, and the like, to be presented on the training monitor 138 or through the speaker (not shown) in order to motivate the trainee 900.

Further, the above setting parameters are merely examples, and there may be other setting parameters. Alternatively, some of the above setting parameters do not have to be provided. In addition, although many of the above setting parameters are for adjusting the difficulty level of the training as described above, parameters irrelevant to the difficulty level may be included. For example, the walking training device 100 can be configured to display an alert icon image on the training monitor 138. Examples of the setting parameters irrelevant to the difficulty level may include parameters for increasing the degree of concentration of the trainee 900 on the training, such as the size and display interval of the alert icon image. In addition, the setting parameters may additionally include time information such as the date and time when the setting operation is performed, or timing information other than the time (for example, information indicating a distinction between the stance phase and the swing phase in one walking cycle).

The above (2) will be described. The detected data of the above (2) may be defined as the training data of the trainee 900 acquired by the walking training device 100 together with the data of the above (1) during execution of the rehabilitation.

The detected data mainly includes sensor data. The sensor data is a sensor value detected by various sensors of the walking training device 100. Examples of the sensor data include the inclination angle of the trunk detected by the posture sensor 217, the load and the inclination angle detected by the handrail sensor 218, and the angle detected by the angle sensor 223. Examples of the sensors that output sensor data include an acceleration sensor, an angular velocity sensor, a position sensor, an optical sensor, a torque sensor, and a weight sensor. Further, an encoder provided in a motor of the winding mechanism, and the like, of the front wire 134, the rear wire 136, and the harness wire 111 may be used as a sensor. Furthermore, the torque sensor (a load cell) of the motor or a current detection unit that detects a driving current value for driving the motor may be used as a sensor.

In addition, the sensor data may include, for example, line-of-sight data acquired by a line-of-sight detection sensor that detects a line of sight. Such line-of-sight data can be acquired by detecting a line of sight using image processing based on an image of at least the eyes of the trainee 900, but also can be acquired by determining the direction (such as upward/downward) of a face based on an image of at least the face of the trainee 900. Such data can also be included in the above detected data. Further, examples of the detected data may include voice data acquired by a voice acquisition unit, such as a microphone that acquires the voices of the trainee 900 or the training staff member 901, text data obtained by analyzing the voice data, or data obtained by analyzing the text data. The voice of the training staff member 901 may include talking to the trainee 900 regarding correction of the walking style, or the like. Further, the sensor data may be data obtained by detecting an electroencephalogram of the trainee 900 using an electroencephalograph, or data obtained by detecting an electroencephalogram of the training staff member 901 using the electroencephalograph.

Further, the line-of-sight detection sensor, a photographing unit that photographs the image, a microphone, and the like, may be provided on the main body of the walking training device 100 or, for example, on a glasses-type wearable terminal to be worn by the trainee 900. The above terminal may be provided with a wireless communication unit that wirelessly communicates data using a wireless communication method, such as Bluetooth^{®}, and the walking training device 100 may also be provided with a wireless communication unit. As such, the walking training device 100 can acquire the data acquired by the wearable terminal via wireless communication. The electroencephalograph may be limited to one with high detection accuracy, but may be configured to be provided on the main body of the walking training device 100, so as to distinguish and detect the electroencephalogram of the trainee 900 and the electroencephalogram of the training staff member 901. However, the electroencephalograph may be provided at a position close to a detection target person, such as the above-described glasses-type wearable terminal (for example, a temple of the glasses).

In addition, the detection unit that acquires the detected data, such as a sensor, is not limited to one described with reference to FIGS. 1 to 3, or the glasses-type wearable terminal, and the like, as illustrated above. For example, a wear on which a wear-type biological sensor and/or a wear-type touch sensor are mounted may be worn by the trainee 900. Here, the wear is not limited to a wear worn on the upper body, and may be a wear worn on the lower body, a top and bottom set, or a wear attached to a part of the brace 110 or the like. Moreover, the wear and the walking training device 100 may be provided with the wireless communication unit, as described above. As such, the walking training device 100 can acquire the data acquired by the wear-type biological sensor or the wear-type touch sensor via wireless communication. The wear-type biological sensor can acquire vital data, such as the heart rate of a wearer. The wear-type touch sensor can acquire data indicating information on a touch on the trainee 900 who is the wearer from the outside, that is, data indicating information on a position where the training staff member 901 touches the trainee 900.

Further, the detected data is not limited to the values indicated by detection signals detected by the various sensors, and the like, and may include a value calculated based on the detection signals from the plurality of sensors, or a statistical value obtained by statistically processing the detection signals from the one or more sensors, and the like. As the statistical value, various statistical values, such as an average value, a maximum value, a minimum value, and a standard deviation value, can be employed. Alternatively, a statistical value based on static statistics, or based on dynamic statistics over a certain time period, such as a day, one round of training, and one walking cycle may be employed.

For example, the sensor data may include the open angle of the knee joint calculated from the angle between the upper leg frame 122 and the lower leg frame 123 detected by the angle sensor 223. Further, the sensor data detected by the angle sensor may include an angular velocity obtained by differentiating the angle. The sensor data detected by the acceleration sensor may be speed obtained by integrating the acceleration or a position obtained by integrating the acceleration twice.

For example, for each rehabilitation performed every day or within one day, the detected data may include an average value, a total value, a maximum value, a minimum value, and a representative value to be described below. Here, examples of the average value may include average speed (a total walking distance/a total walking time) [km/h], an average value of a stride length [cm], and a walking rate indicating the number of steps per minute [steps/min], the walking PCI [beats/m], and fall prevention assistance [%]. The average speed may be, for example, a value calculated from a speed setting value of the treadmill 131, or a value calculated from a driving signal in the treadmill driving unit 211. The stride length refers to a distance between successive ground contacts of a heel of the same foot. The PCI refers to the physiological cost index (a clinical physiological cost index), and the walking PCI indicates energy efficiency during walking. The fall prevention assistance [%] refers to a ratio of the number of times the training staff member 901 assists the trainee 900 to prevent from falling to the number of steps, that is, a ratio of the fall prevention assistance [numbers of times] per one step.

Moreover, examples of the total value here may include a walking time [sec], a walking distance [m], the number of steps [steps], the fall prevention assistance [number of times], body site on which the fall prevention assistance is performed, and the number of times that the fall prevention assistance is performed for each part of the body [number of times]. Further, examples of the maximum value or the minimum value here may include a maximum value or a minimum value of a continuous walking time [sec], a continuous walking distance [m], and the number of continuous steps [steps], and a minimum value of the walking PCI [beats/m] (that is, the longest distance that can be walked per a heartbeat). Examples of the representative value may include a value (representative speed [km/h]) most used as the speed of the treadmill 131.

As described above, the data supplied directly or indirectly from the detection unit, such as various sensors, may be included in the detected data. Further, the detected data may additionally include the time information such as the date and time when the detection is performed, or the timing information other than the time.

Further, the above detected data is merely an example, and there may be other detected data. Alternatively, some of the above detected data does not have to be provided. In other words, when the detected data is employed as the rehabilitation data, the server 500 collects one or more pieces of the detected data.

The above (3) will be described. The data on the trainee 900 (hereinafter, trainee data) indicates, for example, an attribute of the trainee 900. The trainee data may include the age, gender, physique (height, weight, and the like), symptom information, Br. Stage, SIAS, an initial walking FIM, and a latest walking FIM of the trainee 900. In addition, the trainee data may include the name or ID of the trainee 900, taste information indicating a taste of the trainee 900, and personality information indicating a personality thereof. Further, the trainee data may include, as the FIM, an exercise item other than that related to the walking ability and a cognitive item. In other words, the trainee data may include various pieces of data indicating a physical ability of the trainee 900. Moreover, part or all of the trainee data may be referred to as physical information, basic information, trainee feature information, or the like.

Here, the symptom information may include information indicating an initial symptom, the onset of a symptom, and a current symptom, and it is possible to comprehend that the trainee 900 needs rehabilitation mainly due to the symptom included therein. However, a symptom that may not be directly related to rehabilitation may also be included in the symptom information. In addition, the symptom information may include the type (a name of an illness or a disease) of the disease that the trainee 900 suffers, such as a stroke (a cerebrovascular disease) and a spinal cord injury, as well as the site (the injury site), and may include the classification of the disease depending on the type. For example, a stroke may be classified into a cerebral infarction, an intracranial hemorrhage (a cerebral hemorrhage / a subarachnoid hemorrhage), and the like.

Br. Stage refers to the Brunnstrom Stages of Recovery that is a recovery procedure for a hemiplegia, and is classified into six stages starting from observation. The trainee data may include a lower extremity item, which is a main item related to the walking training device 100 among the stages of the Br. Stage. SIAS refers to the Stroke Impairment Assessment Set that is an index that comprehensively assesses a dysfunction caused by a stroke. The SIAS may include a hip joint flexion test (Hip-Flex), a knee extension test (Knee-Ext), and a foot pat test (Foot-Pat). In addition, the SIAS may include a lower extremity tactile sensation test (Touch L/E), a lower extremity position sensation test (Position L/E), an abdominal muscle strength test (Abdominal), and a verticality test (Verticality).

The FIM defines one of assessment methods of assessing activities of daily life (ADL). In the FIM, assessment is performed on a scale of 1 to 7 according to an amount of assistance.

For example, the walking FIM is a general-purpose index indicating the degree of recovery. Moreover, the walking FIM is an index indicating the trainee's movement ability (that is, the walking ability) when the actuator is not used. When the trainee can walk longer than 50 m without an assistant and a brace (an assistive tool), the score is seven, which is the highest score. On the contrary, when the trainee can walk only less than 15 m no matter how much the assistance assists, the score is one, which is the lowest score. In addition, when the trainee can move 50 m with the minimum assistance (the assistance amount of 25% or less), the score is four, and when the trainee can move 50 m with moderate assistance (the assistance amount of 25% or higher), the score is three. Thus, the walking FIM of the trainee 900 gradually increases as the recovery progresses.

Therefore, the walking FIM is an index indicating the degree of recovery of the trainee 900 since the start of rehabilitation. The walking FIM is an index indicating the movement ability, that is, the walking ability, of the trainee 900 when the actuator is not used. In other words, the walking FIM is an important index for comprehending the progress of the rehabilitation of the trainee 900. However, in order to score the walking FIM, the trainee 900 needs to walk 50 m on flat ground with the assistant being nearby. Therefore, it may not be possible to frequently score the walking FIM. Further, as described above, only the assistance level is different between the case where the walking FIM score is three and the case where the walking FIM score is four. In other words, a deviation may occur in scoring the walking FIM depending on who the assistant (a training staff member) is. Moreover, the walking distance for assessment of the walking FIM is not limited to 50 m, and may be, for example, 15 m.

As can be understood from the above, the latest walking FIM used in the walking training device 100 is not only an index indicating the physical ability of the trainee 900 but also an index indicating the degree of recovery of the trainee 900 since the start of the rehabilitation. In other words, the walking FIM is an important index for comprehending the progress of the rehabilitation of the trainee 900. Moreover, an amount of change or rate of change from the initial walking FIM to the latest walking FIM is also an index indicating the degree of recovery. The rate of change may also be referred to as FIM efficiency, and may be a value obtained by, for example, dividing a gain (the amount of change) of the FIM up to the present by a period, such as the number of days for performing rehabilitation, the number of elapsing days indicating the period of rehabilitation, and the number of hospitalization days when the trainee 900 is an inpatient.

In addition, the walking FIM may be comprehended by a score under conditions at the time of assessment, such as a condition in which the trainee wears an assistive device. In this case, information indicating the conditions applied at the time of the assessment may be added to information indicating the walking FIM. Examples of conditions may include the height addition, the assistive device used (for example, the walking assistive device 120, other walking assistive devices, and no assistive device used), a setting for the assistive device used, such as setting of angles for knees and ankles, whether the trainee has walked on flat ground or an incline, at the time of acquiring the information. Moreover, the walking FIM usually means a walking FIM on flat ground, and flat ground walking information thereon may include information such as a maximum distance (the maximum continuous walking distance [m]) that the user has walked at the time of assessment on flat ground walking.

As described above, the trainee data of the above (3) may include index data that includes at least one of the symptom, the physical ability, and the degree of recovery of the trainee 900 in rehabilitation performed by the trainee 900 using the walking training device 100. Moreover, the data that can be included in both the physical ability and the degree of recovery, such as the latest walking FIM, is usually included in one of them but may also be included in both. Further, the same can be applied to all items of rehabilitation data, and data of a certain item may be handled as any one piece or a plurality of pieces of data of the above (1) to (4). In addition, the above trainee data may additionally include time information such as the date and time when the walking FIM is acquired, for example, a measurement date and time of the walking FIM.

The above (4) will be described. Data on the training staff member 901 (hereinafter, staff data) indicates, for example, an attribute of the training staff member 901. The staff data includes the name, ID, age, gender, and physique (height, weight, and the like) of the training staff member 901, the name of the hospital he/she works for, the number of years of experience as a PT or a doctor, and the like. The staff data may include a value obtained by quantifying the timing of assisting the trainee 900 as data on the training staff member.

In addition, when a plurality of training staff members concurrently assist the trainee in rehabilitation, the rehabilitation data may include staff data on a plurality of training staff members. Moreover, each staff data may include information indicating whether the training staff member is a main training staff member, an auxiliary training staff member, a training staff member only performing operations, or a training staff member only supporting the trainee 900 with hands.

Further, the walking training device 100 may be configured to accept an input of a rehabilitation plan on the trainee 900. The data of the rehabilitation plan input as described above may also be included in the rehabilitation data as the staff data on the training staff member 901 who inputs the rehabilitation plan, or as the rehabilitation data included in another category. In addition, to be able to respond to a change in training staff members 901, the walking training device 100 may be configured to accept an input of precautions or an item that needs to be known when assisting the trainee 900 during training in the future. The data input as described above may also be included in the rehabilitation data as the staff data on the training staff member 901 who inputs the above data or as the rehabilitation data included in another category.

The reason that the above data is included in the rehabilitation data is that some training staff members may be able to successfully train the trainee 900 because they saw the precautions and the item that needs to be known from other skilled training staff members. Further, the above staff data may additionally include time information such as the date and time when the data is input, for example, an input date and time of the rehabilitation plan.

### Establishment of Learning Model

The server 500 functions as a learning model establishment device that generates a learning model. Specifically, the server 500 collects the rehabilitation data from the plurality of walking training devices 100. Then, the server 500 accumulates the collected rehabilitation data in the data accumulation unit 520. The server 500 establishes a learned model by performing machine learning based on the rehabilitation data. Specifically, the server 500 generates a learned model that outputs an index indicating the degree of recovery upon receiving an input of the rehabilitation data.

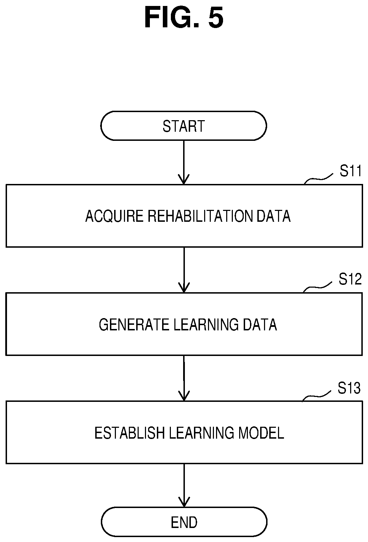

As illustrated in FIG. 4, the control unit 510 of the server 500 includes a data generation unit 510a and a learning unit 510b. FIG. 5 is a flowchart illustrating a method (a learning method) of establishing the learning model in the control unit 510. The control unit 510 acquires the rehabilitation data transmitted from the walking training device 100 (S11). The data generation unit 510a generates the learning data as preprocessing for machine learning (S12). In other words, the data generation unit 510a generates the learning data based on the rehabilitation data accumulated in the data accumulation unit 520. The learning unit 510b establishes a learning model by performing machine learning using the learning data (S13).

The learning unit 510b establishes a learning model that calculates the walking FIM as an index indicating the degree of recovery. For example, the learning unit 510b performs supervised learning. The latest walking FIM actually measured during the flat ground walking of the trainee 900 becomes supervised data (label). In other words, the learning unit 510b prepares learning data including the latest actual measurement value of the walking FIM as supervised data.

For example, in the learning data, the rehabilitation data collected from the walking training device 100 is associated with the actual measurement value of the walking FIM as the supervised data. More specifically, since the rehabilitation data includes the actual measurement value of the walking FIM, the actual measurement value of the walking FIM included in the rehabilitation data becomes the supervised data. Then, immediately after the walking FIM of the trainee 900 is actually measured, the data generation unit 510a uses, as learning data, the rehabilitation data at the time when the trainee 900 performs the walking training. The rehabilitation data used as the learning data is not limited to the rehabilitation data generated immediately after the walking FIM of the trainee 900 is actually measured, and the rehabilitation data of walking training performed within a predetermined period since the time of the actual measurement of the walking FIM may be generated as the learning data. Rehabilitation data collected after a predetermined period has elapsed since the time of the actual measurement of the walking FIM may be set not to be used as the learning data.

The data generation unit 510a prepares a plurality of sets of learning data To this end, the data generation unit 510a prepares the rehabilitation data collected within a predetermined period as one set of learning data. For example, the rehabilitation data collected at one round or one segment of walking training may be prepared as one set of learning data.

One round of walking training is a series of training performed by one trainee 900, and when one round of walking training is ended, a next trainee 900 performs training in the walking training device 100. One round of walking training usually lasts about 20 to 60 minutes. One segment of walking training is one unit in which the trainee 900 walks without stopping as a part of one round of walking training. One round of walking training includes a plurality of segments of walking training. For example, one segment may last about five minutes. Specifically, in one round of walking training, the trainee 900 may perform a 5-minute walking training and then take a 5-minute break. In other words, performing walking training and taking a break may be alternately repeated in one round of walking training. One segment of walking training may be performed for five minutes between two breaks. Of course, periods for one round and one segment of walking training are not particularly limited, and may be appropriately set for each trainee 900.

The data generation unit 510a may prepare the rehabilitation data collected in a period shorter than one segment as the learning data. Alternatively, the data generation unit 510a may prepare the rehabilitation data collected in a period longer than one segment as one set of learning data. Moreover, in the following description, one set of learning data is referred to as a data set for learning (also simply referred to as a data set).

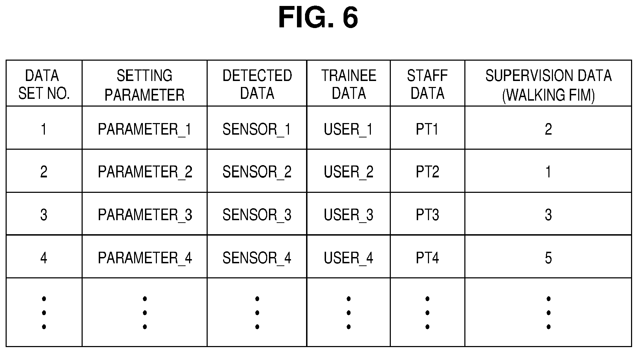

An example of the data set will be described with reference to FIG. 6. FIG. 6 is a table for describing the data set. One data set includes the detected data and the supervised data. Further, one data set may include the setting parameter, the trainee data, and the staff data. In FIG. 6, the setting parameter, the detected data, the trainee data, the staff data, and the supervised data are associated with each other, and constitute one data set. Moreover, as described above, the supervised data is an actual measurement value of the walking FIM measured before the start of the training.

In addition, in FIG. 6, for simplicity of description, each of the setting parameter, the detected data, the trainee data, and the staff data is illustrated as one piece of data (for example, parameter_1), but may actually include a plurality of pieces of data. For example, as described above, the setting parameter may include two or more pieces of data, such as the partial weight load support amount and the vertical position of the handrail 130a. As described above, the trainee data may include two or more pieces of data, such as the initial walking FIM, gender, and age of the trainee 900. As described above, the staff data may include two or more pieces of data, such as the age and gender of the training staff member 901.

Further, the detected data may include data from two or more sensors such as the acceleration sensor, the angular velocity sensor, the position sensor, the optical sensor, the torque sensor, and the weight sensor. Moreover, the data set is not limited to raw data of the detected data, and may include data obtained by performing predetermined processing on the detected data. For example, a feature amount extracted from detected data acquired over a certain time period may be used as the learning data.

For example, the data set may include the maximum value, the minimum value, the relative maximum value, the relative minimum value, the average value, and the like, of the detected data in one segment. The data generation unit 510a may calculate the feature amount from the detected data accumulated in the data accumulation unit 520. Further, the data accumulation unit 520 may accumulate the feature amount. The data accumulation unit 520 may accumulate raw data of the detected data, and the learning model may have a layer that calculates the feature amount.

The learning unit 510b can perform machine learning to solve a regression problem of obtaining a function that derives the walking FIM from the rehabilitation data excluding the walking FIM. Alternatively, since the walking FIM is set on a scale of 1 to 7, the learning unit 510b can perform machine learning to solve a classification problem.

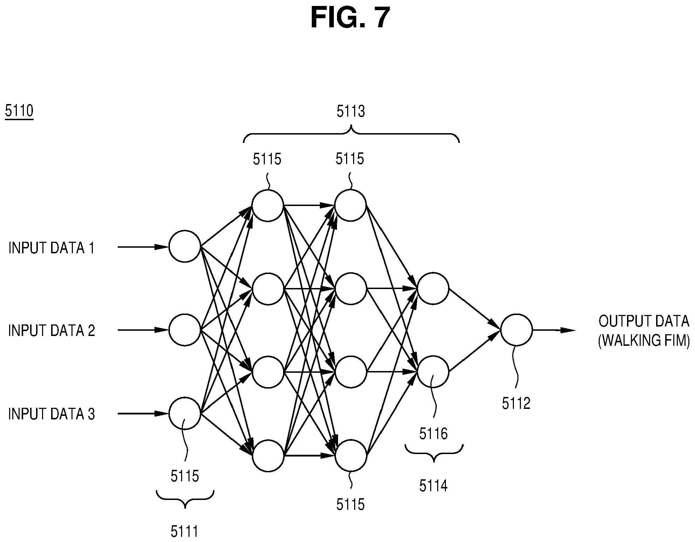

As illustrated in FIG. 7, the learning unit 510b may establish the learning model 5110 using a neural network. The learning model 5110 includes an input layer 5111, an output layer 5112, and an intermediate layer (also referred to as a hidden layer). The intermediate layer 5113 is provided between the input layer 5111 and the output layer 5112. The input layer 5111 has a plurality of nodes 5115, and receives an input of each data included in the data set. The output layer 5112 outputs a predicted value of the walking FIM. The intermediate layer 5113 has a plurality of nodes 5115. Each node has an activation function. Edges that connect the nodes are weighted. The learning model 5110 uses the walking FIM as a target variable and the detected data, and the like, as explanatory variables.

Here, the type of the learning model 5110 to be learned by the learning unit 510b and its algorithm are not limited, but a neural network can be used as the algorithm. Particularly, a deep neural network (DNN) in which the intermediate layer 5113 is multilayered may be used. As the DNN, for example, a feedforward (forward propagation) neural network, such as a multilayer perceptron (MLP) employing the error back propagation algorithm, can be used. Further, the learning model 5110 may be a support vector machine (SVM).

Since the actual measurement value of the walking FIM is input as the supervised data, the learning unit 510b establishes the learning model 5110 such that the predicted value satisfies the actual measurement value. The learning unit 510b may establish the learning model 5110 outputting the predicted value that satisfies the actual measurement value. The learning unit 510b causes the unlearned model to read a large amount of data for learning so as to minimize an error between the actual measurement value and the predicted value. The control unit 510 writes the established learning model 5110 in the model storage unit 521.

The learning unit 510b may establish the learning model using deep learning. Alternatively, the learning unit 510b may establish the learning model using a convolutional neural network (CNN) that performs a convolution calculation. In this case, the learning model 5110 may have a convolution layer, a pooling layer, and the like. The learning unit 510b may establish the learning model using a recurrent neural network (RNN), a long short term memory (LSTM), or the like, that handles time-series data. In an RNN or the like, the intermediate layer of time t-1 is coupled to the intermediate layer of time t. For example, the rehabilitation data collected at a first segment of walking training becomes a data set for learning of time t-1, and the rehabilitation data collected at a second segment becomes a data set for learning of time t. Alternatively, the rehabilitation data collected at a first step in the first segment becomes a data set for learning of time t-1, and the rehabilitation data collected at a second step therein becomes a data set for learning of time t.

Here, examples of input parameters input to the unlearned model and output parameters output from the unlearned model in the learning unit 510b will be described by taking an example in which the learning unit 510b generates the learned model using the MLP. Each of the input parameters corresponds to a node of the input layer, and each of the output parameters corresponds to a node of the output layer (that is, a target variable). Further, as described above, the unlearned model is not limited to a completely unlearned model, but may be a model in which learning is being performed.

Alternatively, one data set may include time-series data, such as the detected data, in order to sequentially input the input data at each time in one segment. In other words, the data set for learning may include log data in a time series. As described above, the feature amount extracted from the log data may be used as the learning data. Further, the data generation unit 510a may perform data processing such that the time-series detected data becomes image data, and use the image data as the learning data. Since a well-known algorithm can be used as the learning method used by the learning unit 510b, detailed description will be omitted.

For example, in walking training of the trainee 900 with a low walking FIM, the driving torque of the joint driving unit 221, the pulling driving unit 214, or the harness driving unit 215 increases. On the contrary, in the walking training of the trainee 900 with a high walking FIM, the driving torque of the above units decreases. Alternatively, in the walking training of the trainee 900 with a low walking FIM, the deviation of the load sensor 222 on the sole increases, and in the walking training of the trainee 900 with a high walking FIM, the deviation decreases.

Therefore, the learning unit 510b can establish the learning model that predicts the walking FIM based on the detected data. Further, the learning unit 510b establishes the learning model using the actual measurement value of the walking FIM as the supervised data. As such, the learning unit 510b can establish the learning model that predicts the walking FIM with high accuracy. When the machine learning is ended, the learning unit 510b writes the learning model 5110 in the model storage unit 521.

Further, in the following description, the learning model written in the model storage unit 521 after the end of the machine learning is also referred to as a learned model. In other words, the learning model in a use stage is also referred to as the learned model. Of course, when new rehabilitation data is accumulated in the data accumulation unit 520, the learned model may be updated by additionally performing machine learning.

The control unit 510 predicts the walking FIM from the newly collected rehabilitation data using the learned model. When the new trainee 900 performs walking training, the walking training device 100 acquires the rehabilitation data. The learned model outputs the walking FIM upon receiving an input of the rehabilitation data. The learned model can predict, with high accuracy, the walking FIM of a trainee 900 for which the walking FIM is not measured, or of a trainee 900 for which a considerable period has elapsed since the walking FIM was measured. Therefore, even when an opportunity to measure the walking FIM is limited, the training staff member 901 can appropriately assess the degree of recovery. Further, it is possible to restrict a deviation in assessment caused by the training staff member 901.

In addition, the learned model predicts the walking FIM as an index including the degree of recovery or the movement ability, but may also predict an index other than the walking FIM. For example, as illustrated in FIG. 7, the learned model may use an output of a node of the intermediate layer 5113 as an index. For example, the output of one node 5116 of the intermediate layer 5114 at the last stage in the intermediate layer 5113 can be used as an index.

In this case as well, since supervised machine learning can be performed using the walking FIM as the supervised data, a learned model with high accuracy can be established. Further, by using the output of the intermediate layer 5114 as an index, it is possible to obtain an index indicating a flat ground walking ability (the degree of recovery) other than the walking FIM. Moreover, the degree of recovery or the movement ability can be assessed on a minute scale having seven or more stages.

### Use of Learning Model

The control unit 510 predicts the value of the walking FIM using the learned model. When a new walking training is performed, the walking training device 100 acquires the rehabilitation data (hereinafter, also referred to as rehabilitation data for assessment). The learned model outputs the walking FIM upon receiving an input of the rehabilitation data for assessment. For example, the rehabilitation data, collected in the walking training of the trainee 900 for which the walking FIM is not measured, or of the trainee 900 for which the considerable period has elapsed since the walking FIM was measured, can be used as the rehabilitation data for assessment. Further, the trainee 900 from which the rehabilitation data for assessment is collected may be the same as or different from the trainee 900 from which the data for learning has collected. In addition, the walking training device 100 that collects the rehabilitation data for assessment may be the same as or different from the walking training device 100 that has collected rehabilitation data as the data for learning.

The learned model can predict, with high accuracy, the walking FIM of the trainee 900 for which the walking FIM is not measured, or of the trainee 900 for which the considerable period has elapsed since the walking FIM was measured. Thus, even when the opportunity to measure the walking FIM is limited, the training staff member 901 can appropriately assess the degree of recovery or the movement ability. Further, it is possible to restrict a deviation in assessment caused by the training staff member 901. The trainee 900 can perform more appropriate walking training. For example, the training staff member 901 can set appropriate setting parameters at the next training based on the predicted value.

When the model storage unit 521 stores the learned model, the server 500 functions as a prediction device that predicts the degree of recovery or the movement ability. Of course, the learned model may be stored in a device, other than the server 500 that has performed the machine learning. In this case, the learning device that performs machine learning is physically different from the prediction device that predicts the degree of recovery or the movement ability using the learned model.

The server 500 may transmit the learned model to the walking training device 100. The walking training device 100 receives a computer program functioning as a learned model via the external communication device 300. A processor provided in the control panel 133 performs the computer program of the learned model. In other words, the detected data from the sensor is used as input data of the learned model. As such, the walking training device 100 can predict the walking FIM during walking training. As described above, when the walking training device 100 stores the learned model, the walking training device 100 functions as a prediction device that predicts the walking FIM.

In addition, in the above description, the control unit 510 established the learning model based on the rehabilitation data collected in the walking training device 100, but may establish the learning model based on the rehabilitation data collected from the rehabilitation support devices other than the walking training device 100. For example, the control unit 510 can use the rehabilitation data collected from a rehabilitation support device for an upper extremity or a rehabilitation support device for balance ability. In this case, the learning model calculates an index indicating a movement ability of the upper extremity or the balance ability.

In summary, the server 500 according to the present embodiment functions as a learning device. The communication IF of the server 500 functions as a data acquisition unit that acquires the rehabilitation data from the rehabilitation support device. Moreover, the rehabilitation support device includes an actuator that assists the rehabilitation movement of the trainee, and a sensor that detects data on the rehabilitation movement assisted by the actuator. The data generation unit 510a generates the rehabilitation data including the detected data corresponding to the detection result by the sensor as the data for learning. By performing machine learning using the data for learning, the learning unit 510b generates the learning model that outputs the index indicating the movement ability of the trainee when the actuator is not used, upon receiving the input of the detected data.

The walking training device 100 operates to output the index indicating a movement ability of the trainee upon receiving the input of the detected data. The walking training device 100 uses the learning model generated by the learning device as the learned model so as to function as the prediction device that predicts the movement ability of the trainee based on the rehabilitation data for assessment. As such, the movement ability can be appropriately assessed, and thus walking training can be more appropriately performed.

### Second Embodiment

In a second embodiment, the server 500 classifies the trainees 900 into a plurality of groups. Then, the server 500 establishes a learning model for each group. Specifically, the server 500 performs clustering for the trainees based on the trainee data. Since the basic configuration and processing of the system according to the second embodiment are the same as those in the first embodiment, description thereof will be properly omitted.

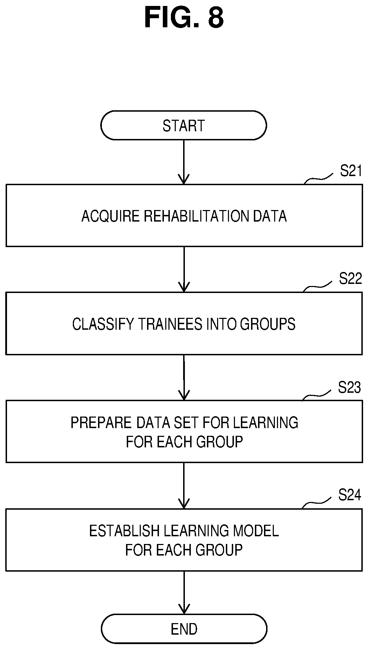

A processing method according to the present embodiment will be described with reference to FIG. 8. FIG. 8 is a flowchart for describing processing in the control unit 510. First, the control unit 510 acquires the rehabilitation data, as in the first embodiment (S21). Then, the control unit 510 classifies the trainees into groups (S22). The values of the initial walking FIM and the SIAS can be used as the training data for classifying the trainees. For example, trainees having the same values of the initial walking FIM and the SIAS may be classified into one group. In addition, the number of groups is not limited as long as it is two or more. Further, the training staff member 901, or the like, may perform part or all of the classification processing.

Moreover, the control unit 510 may establish the learning model for classification using machine learning. In this case, the control unit 510 establishes the learning model for classification using, for example, unsupervised learning. The control unit 510 establishes the learning model using a clustering method, such as k-means clustering. In k-means clustering, when a manager inputs the number of clusters k (an integer of two or greater) in advance, a group including a plurality of trainees 900 is clustered into a k number of clusters. Moreover, the clustering may be non-hierarchical or hierarchical.

The data generation unit 510a prepares the data set for learning for each group (S23). In other words, the data generation unit 510a sets the rehabilitation data of the trainees 900 belonging to the same group as the data set for learning. The learning unit 510b establishes the learning model for each group (S24). In addition, since steps S21, S23, and S24 are the same as those in the first embodiment, description thereof will be omitted.

As described above, the learning unit 510b can establish the learning model for each group. The model storage unit 521 stores the learning model for each group. When using the learning model, the control unit 510 predicts the walking FIM using the learned model of the group to which the trainee 900 belongs. For example, when the server 500 acquires the rehabilitation data for assessment, the server 500 may determine a group of trainees having similar attribute to the trainee 900 using the k-nearest neighbors (KNN) algorithm. Since the control unit 510 can use the learned model suitable for the trainee 900, the control unit 510 can predict the walking FIM with high accuracy.

### Third Embodiment

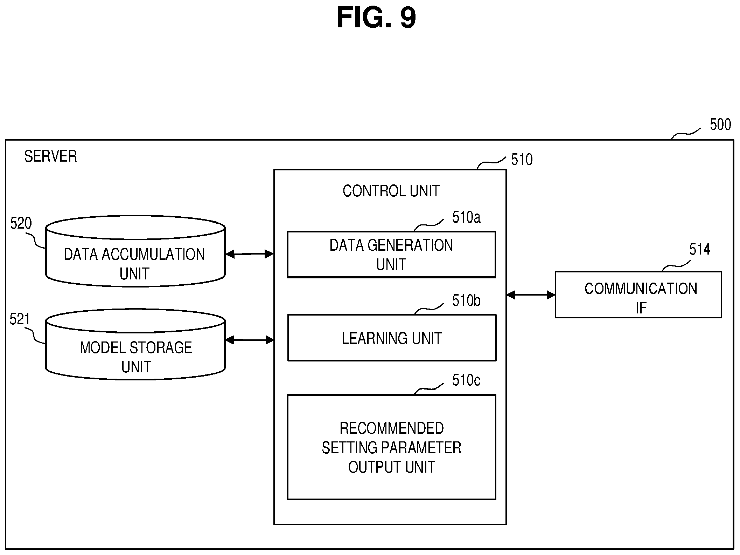

The server 500 according to a third embodiment will be described with reference to FIG. 9. FIG. 9 is a functional block diagram of the server 500. In the present embodiment, a recommended setting parameter output unit 510c is added to the control unit 510. Since the configuration other than the recommended setting parameter output unit 510c and the processing other than a processing therein are the same as those in the first and the second embodiments, description thereof will be omitted.

The recommended setting parameter output unit 510c calculates, for each group, the statistical value of the index indicating a movement ability. The recommended setting parameter output unit 510c outputs a recommended setting parameter based on the statistical value and the predicted value.

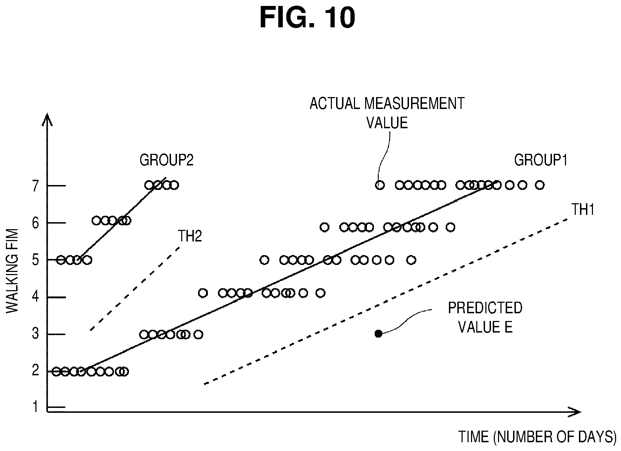

As the statistical value of the index indicating the movement ability, the rate of change of the walking FIM, that is, the FIM efficiency, can be used. FIG. 10 is a graph illustrating the rate of change of the walking FIM, in which the horizontal axis represents time (for example, the number of hospitalization days or the number of training days), and the vertical axis represents the walking FIM. FIG. 10 illustrates a graph of group 1 having a low initial walking FIM and group 2 having a high initial walking FIM. Specifically, the initial walking FIM of group 1 is two, and the initial walking FIM of group 2 is based on a data pair [the number of days and the walking FIM] extracted from the rehabilitation data of walking training performed in the past by the plurality of trainees 900. In FIG. 10, white circles represent the data of the actual measurement values.

For example, the recommended setting parameter output unit 510c obtains an approximate function of the rate of change by performing the method of least squares, or the like, on a plurality of data pairs. Then, an approximate function of the rate of change of the walking FIM is used as a statistical value. Moreover, the statistical value may be obtained from only the actual measurement value of the walking FIM, only the predicted value, or both the actual measurement value and the predicted value. In FIG. 10, an approximate straight line of the rate of change of the walking FIM is used as the statistical value, but an approximate curve, such as a polynomial, may be used as the statistical value.

When the predicted value of the walking FIM is significantly lower than the statistical value, it is presumed that the walking training is not effective. Therefore, the recommended setting parameter output unit 510c may present the recommended setting parameters so as to improve the setting parameter of the walking training. For example, the recommended setting parameter output unit 510c compares the predicted value with a threshold set from the statistical value set based on the number of training days of the trainee 900. For example, a predetermined ratio with respect to the statistical value can be the threshold. In FIG. 10, a threshold TH1 is set from the statistical value of group 1 and a threshold TH2 is set from the statistical value of group 2. Each of the thresholds TH1, TH2 is illustrated as a linear function that gradually increases with the number of days. When the group and the number of days are determined, the recommended setting parameter output unit 510c can obtain the threshold. The thresholds TH1, TH2 indicate a target recovery degree (also referred to as a target movement ability) with respect to the number of training days in each group.

In FIG. 10, a predicted value E is a predicted value of the walking FIM of the trainee 900 belonging to group 1. Since the predicted value E of the walking FIM is less than the threshold TH1 set from the statistical value, the recommended setting parameter output unit 510c determines that walking training is not effective, and outputs a setting parameter effective for training. In other words, when the predicted value does not reach the target recovery degree, the recommended setting parameter output unit 510c outputs the recommended setting parameter.

The control unit 510 transmits the recommended setting parameter to the walking training device 100 via the communication IF 514. Upon receiving the recommended setting parameter, the walking training device 100 displays the recommended setting parameter on the management monitor 139. Upon checking the management monitor 139, the training staff member 901 changes or adjusts the setting parameter based on the recommended setting parameter. Alternatively, the walking training device 100 may automatically set the recommended setting parameter. The recommended setting parameter is not limited to any type of setting parameter, and may be one or more types.

The recommended setting parameter output unit 510c can set the recommended setting parameter based on a setting parameter used in past walking training. For example, the recommended setting parameter output unit 510c extracts, in advance, a trainee having a high rate of change in the walking FIM, that is, a trainee who has effectively performed walking training, as a recommended trainee from the group. In the rehabilitation data collected from the walking training of the recommended trainee, the setting parameter when the number of training days and the value of the walking FIM are close to those of the trainee 900 is set as the recommended setting parameter.

Alternatively, a plurality of recommended trainees in one group may be set in advance. When the plurality of recommended trainees are set, one recommended trainee similar to the trainee 900 may be selected from among the plurality of recommended trainees. Alternatively, a representative value, such as an average value or a median, of the setting parameters of the plurality of recommended trainees may be used as the recommended setting parameter.

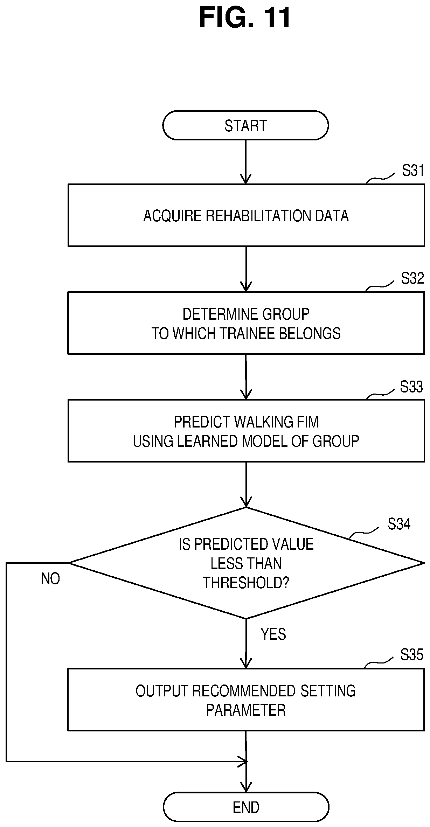

The processing in the server 500 will be described with reference to FIG. 11. FIG. 11 is a flowchart illustrating processing of obtaining the recommended setting parameter in the server 500. In addition, part or all of the processing illustrated in FIG. 11 may be performed in the walking training device 100.

First, the server 500 acquires the latest rehabilitation data (S31). Then, the recommended setting parameter output unit 510c determines a group to which a trainee 900 will belong based on the trainee data included in the rehabilitation data (S32). For example, the recommended setting parameter output unit 510c can determine a group of trainees having similar attribute to the trainee of the latest rehabilitation data by referring to the past rehabilitation data accumulated in the data accumulation unit 520.

The recommended setting parameter output unit 510c determines the group to which the trainee will belong using the KNN algorithm. The recommended setting parameter output unit 510c extracts, for example, a feature amount vector that includes a plurality of feature amounts from the trainee data. The recommended setting parameter output unit 510c determines the k number of trainees who are closest to the feature amount vector among the past trainees. Thereafter, the recommended setting parameter output unit 510c determines a group including the most trainees out of the k number of trainees, as a group to which the trainee at the time of assessment belongs.

The recommended setting parameter output unit 510c predicts the walking FIM using the learned model of the determined group (S33). The recommended setting parameter output unit 510c inputs, to the learned model, the rehabilitation data including the detected data, and the like. As such, the learned model outputs the predicted value of the walking FIM. Then, the recommended setting parameter output unit 510c determines whether the predicted value is less than the threshold (S34). As described above, the threshold is a value set based on the statistical value for each group. Thereafter, the recommended setting parameter output unit 510c compares the predicted value with the threshold based on the number of most recent training days, and the like, of the trainee.

When the predicted value is not less than the threshold (NO in S34), the recommended setting parameter output unit 510c ends the processing as it is. When the predicted value is less than the threshold (YES in S34), the recommended setting parameter output unit 510c outputs the recommended setting parameter (S35). As described above, the server 500 transmits the recommended setting parameter to the walking training device 100. Moreover, the recommended setting parameter is set based on past rehabilitation data in the group.

As described above, it is possible to perform training with a more appropriate setting parameter. Therefore, since training can be performed more effectively, performance of the walking training can be improved. The recommended setting parameter output unit 510c may output the recommended setting parameter in real time during training. Alternatively, the recommended setting parameter output unit 510c may output the recommended setting parameter whenever one segment of walking training or one day's walking training is performed.

Further, the learning unit 510b may generate the learning model that outputs the recommended setting parameters. For example, upon receiving inputs of the number of training days and trainee data on the group, the learning model outputs the recommended setting parameters.

### Fourth Embodiment

Before describing the present embodiment, an abnormal walking pattern in walking training will be described. The present inventors have obtained the knowledge that the abnormal walking observed in hemiplegic patients has at least seven patterns. In other words, it has been found that if an abnormal walking criterion is determined for each pattern, when the trainee's walking style meets any of the abnormal walking criteria, the trainee's walking movement can be assessed as abnormal. Therefore, in the walking training device 100 according to the present embodiment, the walking assessment unit 210a assesses whether the walking movement is abnormal by comparing the movement amount of each paralyzed part with each abnormal walking criterion. Hereinafter, each abnormal walking criterion and an assessment method thereof will be described.

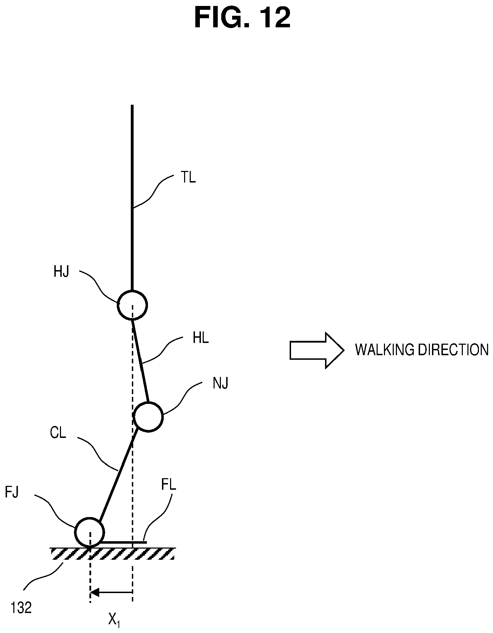

FIG. 12 is a diagram describing a first abnormal walking criterion. FIG. 12 is a schematic diagram of a case in which the paralyzed part that is the lower part of the injured leg is observed from the side with respect to the walking direction. In order from the top, the trunk TL, the hip joint HJ, the upper leg HL, the knee joint NJ, the lower leg CL, the ankle joint FJ, and the foot FL are represented. Further, in the present embodiment, the "leg" and the "leg part" are used as terms indicating the entire lower part than the hip joint HJ, and the "foot" and the "foot part" are used as terms indicating the part from the ankle to the toe.

In order to determine whether the walking movement meets the first abnormal walking criterion, the total control unit 210 detects, as a first movement amount accompanied with the walking movement, a distance X₁ between the hip joint HJ and the ankle joint FJ in the walking direction when the injured leg contacts the ground after the swing phase. In a case of normal walking with the healthy leg, the contacting point after the swing phase is ahead of (forward) the hip joint HJ in the walking direction. However, in a case of walking with the injured leg, since the injured leg cannot be sufficiently swung out and thus cannot sufficiently moved forward, the injured leg contacts the ground slightly ahead of the hip joint HJ or behind the hip joint HJ.

Therefore, "less than a criterion distance X_{c1}" is set as the first abnormal walking criterion. When the distance X₁ detected in the walking movement is less than the criterion distance X_{c1}, it is determined that the walking movement is abnormal. The total control unit 210 acquires the detection signal from the load sensor 222 and the image data from the camera 140, and detects the distance X₁ at the end of the swing phase using the acquired signal and data. For example, when X_{c1} is set to 20 cm (= 20 cm ahead of the hip joint HJ) and the detected distance X₁ is 10 cm or -5 cm, the walking assessment unit 210a assesses the walking movement as abnormal. The criterion distance X_{c1} may be changed according to the trainee's physique, the degree of progress of rehabilitation, or the like.

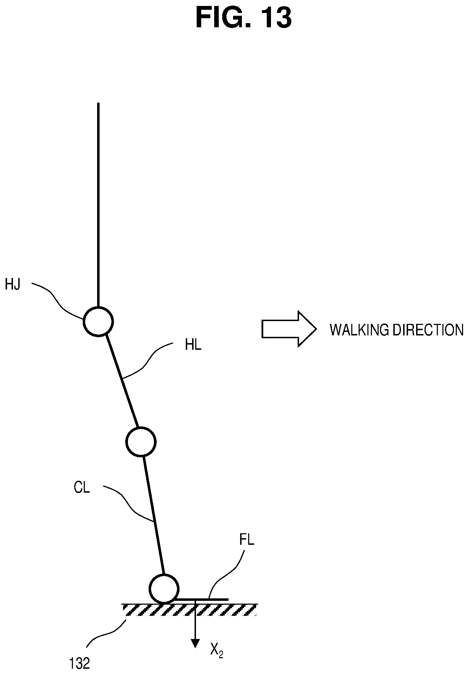

FIG. 13 is a diagram describing a second abnormal walking criterion. FIG. 13 is a schematic diagram of the case in which the paralyzed part that is the lower body of the injured leg is observed from the side in the walking direction, and each body part is represented similarly to FIG. 12.

In order to determine whether the walking movement meets the second abnormal walking criterion, the total control unit 210 detects, as a second movement amount accompanied with the walking movement, a load X₂ on the sole during the swing phase of the injured leg. In the case of normal walking of the healthy leg, the sole does not contact the ground during the swing phase. However, in the case of walking of the injured leg, since strength to lift the entire leg is not sufficient, the walking may become so-called shuffling, in which the leg is pushed forward while the sole is in contact with the ground.

Therefore, "greater than a criterion load X_{c2}" is set as the second abnormal walking criterion. When the load X₂ detected in the walking movement is greater than the criterion load X_{c2}, it is determined that the walking movement is abnormal. The total control unit 210 acquires the detection signal from the load sensor 222 and the image data from the camera 140, and detects the load X₂ during the swing phase using the acquired signal and data. Usually, X_{c2} is set to zero. In other words, when any load on the sole is detected during the swing phase, the walking assessment unit 210a assesses the walking movement as abnormal. However, the criterion load X_{c2} may be set to, for example, 10N to allow a bit of grounding according to the degree of progress of rehabilitation. Further, an integrated load during the swing phase may be used as the criterion value.

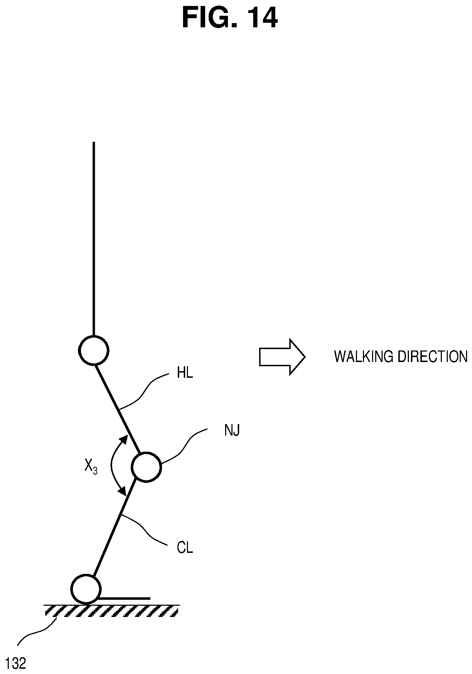

FIG. 14 is a diagram describing a third abnormal walking criterion. FIG. 14 is a schematic diagram of the case in which the paralyzed part that is the lower body of the injured leg is observed from the side with respect to the walking direction, and each body part is represented similarly to FIG. 12.

In order to determine whether the walking movement meets the third abnormal walking criterion, the total control unit 210 detects, as a third movement amount accompanied with the walking movement, a flexion angle X₃ of the knee joint NJ during the stance phase of the injured leg. In the case of normal walking of the healthy leg, the knee joint NJ is not significantly flexed during the stance phase. However, in the case of walking of the injured leg, since the knee joint NJ does not have sufficient strength to support the upper body, the knee j oint NJ may be significantly flexed during the stance phase. In some cases, a so-called knee giving-way occurs.

Therefore, "less than a criterion angle X_{c3}" is set as the third abnormal walking criterion. When the flexion angle X₃ detected in the walking movement is less than the criterion angle X_{c3}, it is determined that the walking movement is abnormal. The total control unit 210 acquires the detection signal from the angle sensor 223 and the image data from the camera 140, and detects the flexion angle X₃ during the stance phase using the acquired signal and data. For example, when X_{c3} is set to 165 degrees and a detected flexion angle X₃ is 140 degrees, the walking assessment unit 210a assesses the walking movement as abnormal. In addition, when the flexion angles X₃ are continuously detected during the stance phase, if the flexion angle X₃ is less than the criterion angle X_{c3} at least once, the walking assessment unit 210a assesses the walking movement as abnormal. The criterion angle X_{c3} may be changed according to the age, the degree of progress of rehabilitation, or the like, of the trainee.

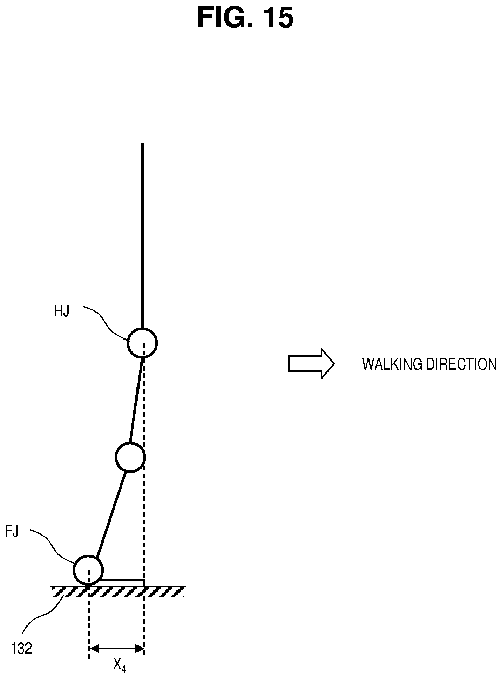

FIG. 15 is a diagram describing a fourth abnormal walking criterion. FIG. 15 is a schematic diagram in which the paralyzed part that is the lower body of the affected leg is observed from the side with respect to the walking direction, and each body part is represented similarly to FIG. 12.

In order to determine whether the walking movement meets the fourth abnormal walking criterion, the total control unit 210 detects, as a fourth movement amount accompanied with the walking movement, a distance X₄ between the hip joint HJ and the ankle joint FJ in the walking direction at the start of swinging when the injured leg switches from the stance phase to the swing phase. In a case of normal walking of a healthy person, the ankle joint FJ is located slightly behind the hip joint HJ at the start of the swing phase. However, in a case of walking of a paralyzed patient, since he/she cannot freely shift the weight of his/her upper body, the swinging may start before the ankle joint FJ is sufficiently far from the hip joint HJ.

Therefore, "equal to or greater than a criterion distance X_{c4}" is set as the fourth abnormal walking criterion. When the distance X₄ detected in the walking movement is equal to or greater than the criterion distance X_{c4}, it is determined that the walking movement is abnormal. The total control unit 210 acquires the detection signal from the load sensor 222 and the image data from the camera 140, and detects the distance X₄ at the start of the swing phase when the injured leg switches from the stance phase to the swing phase, using the acquired signal and data. For example, when X_{c4} is set to -20 cm (= 20 cm backward from the hip joint HJ) and the detected distance X₄ is -10 cm (= 10 cm backward from the hip j oint HJ) or 5 cm (= 5 cm forward from the hip j oint HJ), the walking assessment unit 210a assesses the walking movement as abnormal. The criterion distance X_{c4} may be changed according to the physique, the degree of progress of rehabilitation, or the like, of the trainee.

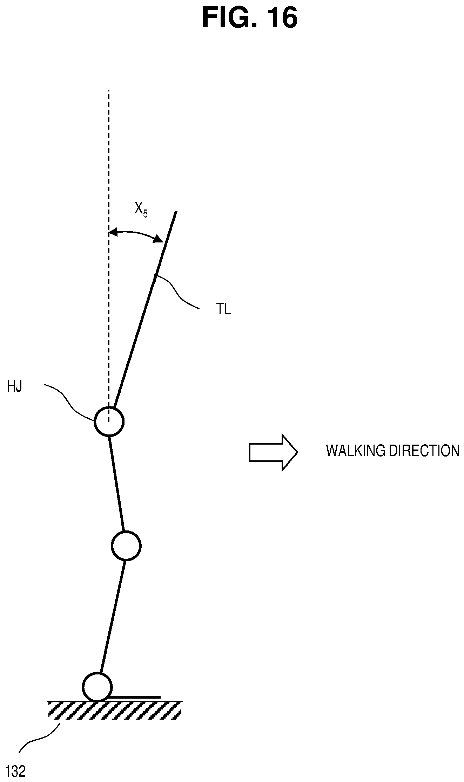

FIG. 16 is a diagram describing a fifth abnormal walking criterion. FIG. 16 is a schematic diagram of the case in which the paralyzed part that is the lower body of the injured leg is observed from the side with respect to the walking direction, and each body part is represented similarly to FIG. 12.

In order to determine whether the walking movement meets the fifth abnormal walking criterion, the total control unit 210 detects, as a fifth movement amount accompanied with the walking movement, a forward inclination angle X₅ of the trunk TL during the stance phase of the injured leg. In the case of normal walking of a healthy person, the trunk TL during the stance phase is slightly inclined forward with respect to a vertical line passing through the hip joint HJ. However, in the case of walking of a paralyzed patient, since he/she tries to protect the lower body, the trunk TL may be significantly inclined forward with respect to the vertical line passing through the hip joint HJ.

Therefore, "equal to or greater than the criterion angle X_{c5}" is set as the fifth abnormal walking criterion. When the forward inclination angle X₅ detected in the walking movement is equal to or greater than the criterion angle X_{c5}, it is determined that the walking movement is abnormal. The total control unit 210 acquires the detection signal from the posture sensor 217 and the image data from the camera 140, and detects the inclination angle X₅ during the stance phase using the acquired signal and data. For example, when X_{c5} is set to 10 degrees and the detected inclination angle X₅ is 30 degrees, the walking assessment unit 210a assesses the walking movement as abnormal. Moreover, when the inclination angles X₅ are continuously detected during the stance phase, if the inclination angle X₅ is equal to or greater than the criterion angle X_{c5} at least once, the walking assessment unit 210a assesses the walking movement as abnormal. The criterion angle X_{c5} may be changed according to the age, the degree of progress of rehabilitation, or the like, of the trainee.

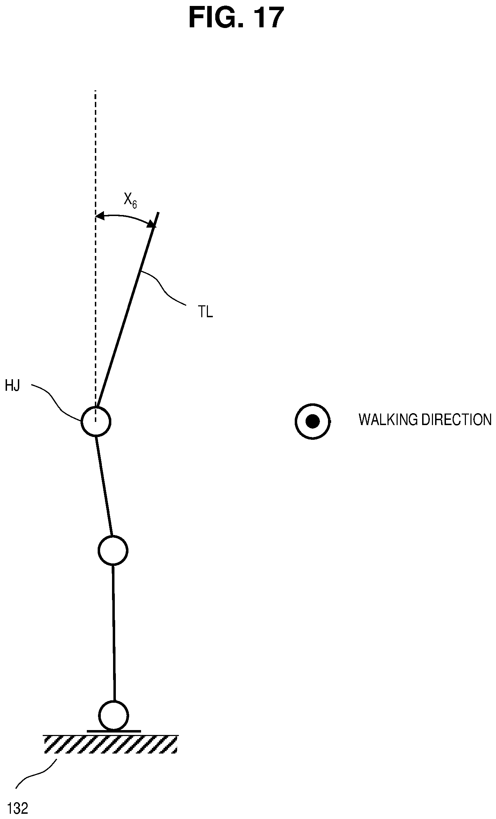

FIG. 17 is a diagram describing a sixth abnormal walking criterion. FIG. 17 is a schematic diagram of the case in which the paralyzed part that is the lower body of the injured leg is observed from the front in the walking direction, and each body part is represented in the same manner as in FIG. 12.

In order to determine whether the walking movement meets the sixth abnormal walking criterion, the total control unit 210 detects, as a sixth movement amount accompanied with the walking movement, an inclination angle X₆ of the trunk TL toward the injured leg during the stance phase of the injured leg. In the case of normal walking of a healthy person, the trunk TL during the stance phase sways hardly from side to side with respect to the vertical line passing through the hip joint HJ. However, in the case of walking of a paralyzed patient, due to fear of putting the weight to the injured leg, the trunk TL may be significantly inclined forward toward the injured leg with respect to the vertical line passing through the hip joint HJ.

Therefore, "equal to or greater than a criterion angle X_{c6}" is set as the sixth abnormal walking criterion. When the inclination angle X₆ toward the injured leg detected in the walking movement is equal to or greater than the criterion angle Xₑ₆, it is determined that the walking movement is abnormal. The total control unit 210 acquires the detection signal from the posture sensor 217 and the image data from the camera 140, and detects the inclination angle X₆ during the stance phase using the acquired signal and data. For example, when X_{c6} is set to 10 degrees and the detected inclination angle X₆ is 20 degrees, the walking assessment unit 210a assesses the walking movement as abnormal. Further, when the inclination angles X₆ are continuously detected during the stance phase, if the inclination angle X₆ is equal to or greater than the criterion angle X_{c6} at least once, the walking assessment unit 210a assesses the walking movement as abnormal. The criterion angle X_{c6} may be changed according to the age, the degree of progress of rehabilitation, or the like, of the trainee.

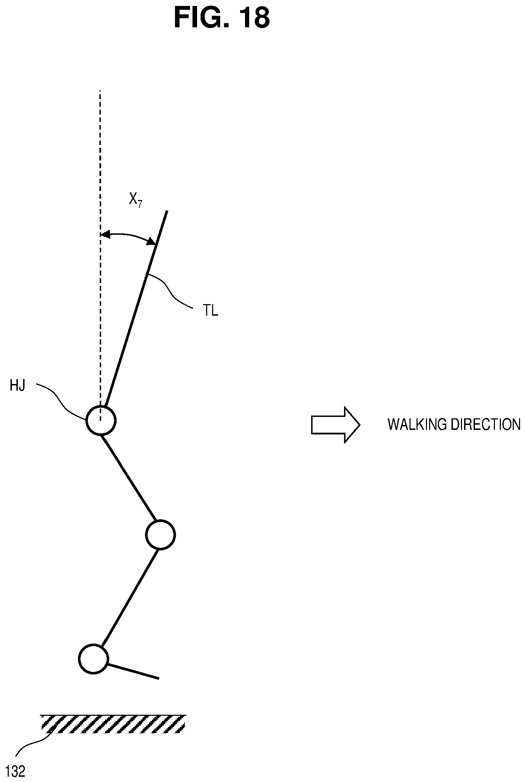

FIG. 18 is a diagram describing a seventh abnormal walking criterion. FIG. 18 is a schematic diagram of the case in which the paralyzed part that is the lower body of the injured leg is observed from the side with respect to the walking direction, and each body part is represented similarly to FIG. 12.

In order to determine whether the walking movement meets the seventh abnormal walking criterion, the total control unit 210 detects, as a seventh movement amount accompanied with the walking movement, a forward inclination angle X₇ of the trunk TL during the swing phase of the injured leg. In the case of normal walking of a healthy person, the trunk TL is slightly inclined forward with respect to the vertical line passing through the hip j oint HJ during the swing phase. However, in the case of walking of a paralyzed patient, since he/she cannot freely shift the weight of the upper body and the upper body bends backward, the trunk TL may be inclined backward with respect to the vertical line passing through the hip joint HJ.

Therefore, "less than the criterion angle X_{c7}" is set as the seventh abnormal walking criterion. When the forward inclination angle X₇ detected in the walking movement is less than the criterion angle X_{c7}, it is determined that the walking movement is abnormal. The total control unit 210 acquires the detection signal from the posture sensor 217 and the image data from the camera 140, and detects the inclination angle X₇ during the swing phase using the acquired signal and data. For example, when X_{c7} is set to -5 degrees (= inclined by 5 degrees backward) and the detected inclination angle X₇ is -20 degrees, the walking assessment unit 210a assesses the walking movement as abnormal. In addition, when the inclination angles X₇ are continuously detected during the swing phase, if the inclination angle X₇ is less than the criterion angle X_{c7} at least once, the walking assessment unit 210a assesses the walking movement as abnormal. The criterion angle X_{c7} may be changed according to the age, the degree of progress of rehabilitation, or the like, of the trainee.

The seven abnormal walking criteria have been described above, but other abnormal walking criteria may be added. When determining the abnormal walking criteria, it is important to determine a plurality of criteria rather than a single criterion. In such a case, the abnormal walking criteria may include at least two or more criteria for the movement amount of different portions of the paralyzed part, or two or more criteria for the movement amount of the same portion of the paralyzed part in different directions.

Here, the two or more criteria for the movement amounts of the different portions may be selected from among the criteria for the movement amount of the trunk, criteria for the movement amount of the knee j oint, and the criteria for the movement amount of the part from the ankle to the tip of the foot. In the above example, the criterion for the movement amount of the trunk relates to the fifth, sixth, and seventh criteria, the criterion for the movement amount of the knee joint relates to the third criterion, and the criterion for the movement amount of the foot relates to the first, second and fourth criteria. It has been found through experiments that when a focused movement amounts are selected as above, in the case in which an actual walking style is to be assessed as abnormal in many cases, even if the walking style is not assessed as abnormal in terms of one of the movement amounts, it is assessed as abnormal in terms of the other movement amounts.

Further, two or more criteria for the movement amounts of the same portion of the paralyzed part in different directions may include a criterion for the movement amount of the trunk in the walking direction and a criterion for the movement amount of the trunk in the direction perpendicular to the walking direction. An example of the above description corresponds to the relationship between any of the fifth and seventh criteria, and the sixth criterion. As such, it has also been found through experiments that when focused movement amounts are combined together, in the case in which an actual walking style is to be assessed as abnormal, in many cases, even if the walking style is not assessed as abnormal in terms of one of the movement amounts, it is assessed as abnormal in terms of the other movement amounts.

In addition, it has been found that the abnormal walking criteria should be different between the swing phase and the stance phase of the injured leg. The first and the fourth criteria relate to the same part in the same direction, but one is focused on the time at which the leg switches from the swing phase to the stance phase, and the other is focused on the time at which the leg switches from the stance phase to the swing phase. Similarly, the fifth and the seventh criteria relate to the same part in the same direction, but one focuses on the stance phase and the other focuses on the swing phase. In other words, even when the movement amounts relate to the same part in the same direction, they may be assessed as feature amounts having walking movements different from each other if an observation time is different.

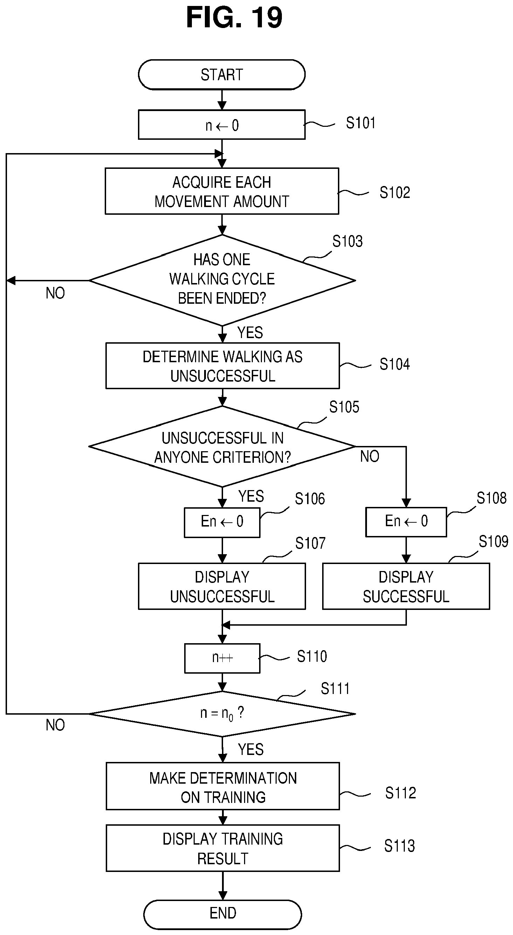

Next, a processing operation of the walking training device 100 will be described. FIG. 19 is a flowchart illustrating the processing operation. The flow starts when the trainee 900 or the operator selects a training menu, and then a series of training programs are started.

In step S101, the total control unit 210 resets a walking cycle counter n. Then, the total control unit 210 starts the rotation of the belt 132 by driving the treadmill driving unit 211, and concurrently performs walking assistance for the trainee 900 by driving the pulling driving unit 214 and the joint driving unit 221 according to a set adjustment value. When the trainee 900 starts a walking movement, in step S102, the total control unit 210 acquires each movement amount accompanied with the walking movement. More specifically, the image processing unit 216 analyzes the image signal acquired from the camera 140, or the total control unit 210 acquires the detection signals from the posture sensor 217, the load sensor 222, and the angle sensor 223 and converts the detection signals into movement amounts.

In step S103, the total control unit 210 determines whether one walking cycle has been ended. The assessment of abnormal walking may be performed for each step of the injured leg, but in the present embodiment, the assessment is performed in one cycle of one step of the injured leg and the following one step of the healthy leg. Therefore, upon determining that the one walking cycle has been ended, the total control unit 210 proceeds to step S104 to perform the assessment. When determining that the one walking cycle has not been ended, the total control unit 210 returns to step S102 and continues acquiring each movement amount.

In step S104, the walking assessment unit 210a of the total control unit 210 assesses abnormal walking. Specifically, the walking assessment unit 210a aggregates the movement amount of each part, each direction, and each period in the walking movement, and checks whether the movement amount meets each abnormal walking criterion described above. Upon assessing the walking movement as abnormal, the walking assessment unit 210a determines that the walking movement is unsuccessful walking. In step S105, the walking assessment unit 210a determines whether the movement amount meets any one of the above abnormal walking criteria. Upon determining that the movement amount meets any one of the abnormal walking criteria, the walking assessment unit 210a proceeds to step S106, and substitutes zero for an nth step assessment variable En. Then, the total control unit 210 displays an indication that the nth step is unsuccessful walking on the training monitor 138 and the management monitor 139 via the display control unit 213. On the other hand, in step S105, upon determining that the movement amount does not meet any of the abnormal walking criteria, the walking assessment unit 210a proceeds to step S108, and substitutes one for the nth step assessment variable En. Thereafter, the total control unit 210 displays an indication that the step is successful walking on the training monitor 138 and the management monitor 139 via the display control unit 213.

Here, when the indication that the walking movement is unsuccessful walking is displayed in real time during training, the training monitor 138 and the management monitor 139 may display a simple and singular indication without indicating which abnormal walking criteria are met. Alternatively, the training monitor 138 and the management monitor 139 may indicate whether each of the abnormal walking criteria has been met. In this case, the above assessment variable En is prepared for each pattern. In order to present the indication whether a walking movement is failure walking, a buzzer sound, blinking light, or the like can be used instead of the management monitor 139. In this case, the sound or light may also be presented to the trainee 900 in a simple and singular manner. As described above, the management monitor 139 which presents the indication that the walking movement is the unsuccessful walking, a device that emits the sound or light, and the like, function as a presentation unit that presents information on the assessment performed by the walking assessment unit 210a.

Upon completing the failure display in step S107 or the success display in step S109, the total control unit 210 proceeds to step S110, and increases the walking cycle counter n. Then, in step S111, the total control unit 210 determines whether the walking cycle counter n has reached the number of walking cycles n₀ scheduled in a series of walking training programs. Upon determining that the walking cycle counter n has not reached the number of walking cycles n₀, the total control unit 210 returns to step S102 and continues controlling the walking training. Upon determining that the walking cycle counter has reached the number of walking cycles n₀, the total control unit 210 proceeds to step S112.

In step S112, the training determination unit 210b of the total control unit 210 aggregates the assessment results of the series of walking training programs in which the user walks continuously, and makes a determination to display the degree of success of the walking training. Specifically, the training determination unit 210b derives the assessment results of the training by calculating the ratio of the number of unsuccessful walking steps to the total number of walking steps of the injured leg, or assessing the number of times of fall prevention operations in which the harness driving unit 215 is operated. The total control unit 210 ends the series of processes after the training determination unit 210b displays the determination results on the training monitor 138 and the management monitor 139 via the display control unit 213 in step S113.

The walking assessment unit 210a makes an abnormal walking determination based on the preset abnormal walking criteria. In other words, when the movement amount meets an abnormal walking criterion, the walking assessment unit 210a determines that the walking movement corresponds to an abnormal walking pattern corresponding to the abnormal walking criterion. On the other hand, when the movement amount does not meet an abnormal walking criterion, the walking assessment unit 210a determines that the walking movement does not correspond to the abnormal walking pattern corresponding to the abnormal walking criterion. The walking training device 100 records, as detected data, the determination results on whether the walking movement corresponds to the abnormal walking pattern.

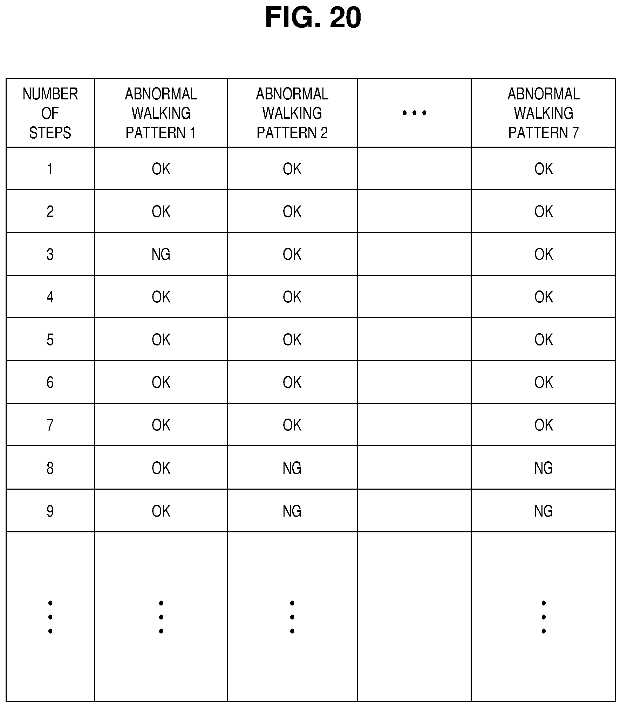

FIG. 20 is a table illustrating the determination results of abnormal walking patterns. As illustrated in FIG. 20, the detected data includes the determination result of whether each walking step corresponds to the seven abnormal walking patterns. In FIG. 20, when a walking step meets an abnormal walking criterion, the result is NG indicating that the walking step corresponds to the abnormal walking pattern, and when the walking step does not satisfy an abnormal walking criterion, the result is OK indicating that the walking step does not correspond to the abnormal walking pattern. Of course, one walking step may correspond to two or more abnormal walking patterns. In the above description, the number of abnormal walking patterns is seven, but may be less than seven, or eight or more. In other words, the number of abnormal walking patterns may be one or more. Further, the walking training device 100 may store the determination results and the moving image of the walking movement in association with each other for each step.

The walking training device 100 transmits, to the server 500, as the detected data, determination results on whether a walking step corresponds to the abnormal walking patterns as illustrated in FIG. 20. The server 500 collects, as part of the rehabilitation data, the determination results on whether a walking step corresponds to the abnormal walking patterns. Moreover, the walking training device 100 may transmit data for determination (for example, the distance Xi) as the detected data, instead of the determination results. Then, the server 500 may perform the abnormal walking determination.

The control unit 510 performs machine learning using the determination results of the abnormal walking determination. Specifically, the data generation unit 510a generates data sets before and after the timing at which the determination results of the abnormal walking determination change. The learning unit 510b performs machine learning using the RNN, by using the data sets generated before and after the change of the determination results.

For example, in the determination results illustrated in FIG. 20, the first to second steps do not correspond to any abnormal walking pattern, the third step corresponds to abnormal walking pattern 1, and the fourth to seventh steps do not correspond to any abnormal walking pattern. In addition, the eighth to ninth steps correspond to the second and seventh abnormal walking patterns, and these are the same. Therefore, the data generation unit 510a sets the data of the second, third, fourth, seventh, and eighth steps as one data set, respectively. In other words, since the fourth to the seventh steps are not abnormal walking and thus no change occurs, the data generation unit 510a excludes the data of the fifth and the sixth steps from the data set for learning. As such, a data set with a large amount of change can be prepared. Furthermore, since a data set with a small amount of change can be excluded, it is possible to restrain a load of the calculation processing.

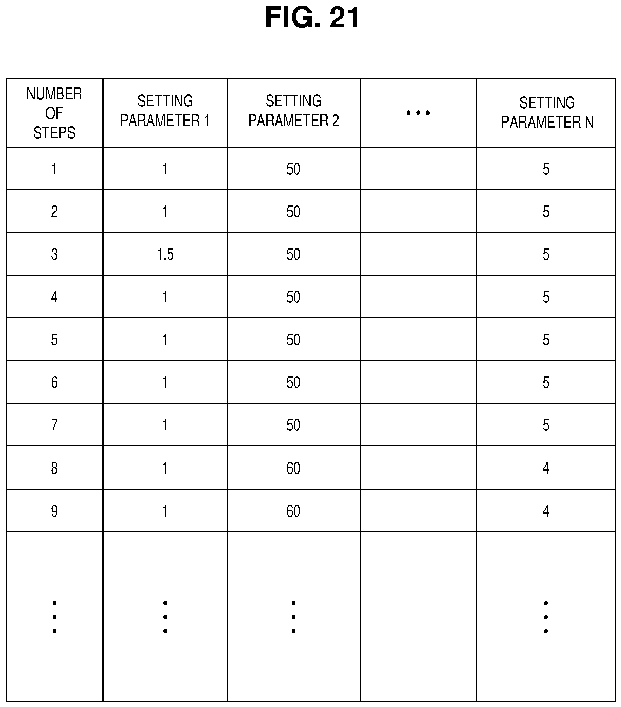

The data generation unit 510a may generate the data set before and after the timing at which the setting parameter changes in addition to a case where a change occurs in the detected data. For example, when the training staff member 901 changes the setting parameter, such as the assisting level in the walking training device 100 during one segment of walking training, the total control unit 210 records a change in the setting parameter for each step. For example, as illustrated in FIG. 21, the total control unit 210 records the value of the setting parameter for each step.

Setting parameters 1 to N are different from each other. For example, setting parameter 1 indicates treadmill speed [km/h], setting parameter 2 indicates a partial weight load reduction amount [%], and the like. The walking training device 100 transmits, to the server 500, the setting value of the setting parameter for each step. Then, the server 500 generates the data set before and after the timing at which the setting parameter is changed, as in the case of the determination results of the setting parameter.

Moreover, in the above example, the data generation unit 510a generates one data set for each step, but may set data collected for a plurality of steps as one data set. For example, the data generation unit 510a may set data for a plurality of steps as one data set. Alternatively, the data generation unit 510a may set data for one segment of walking training as one data set.

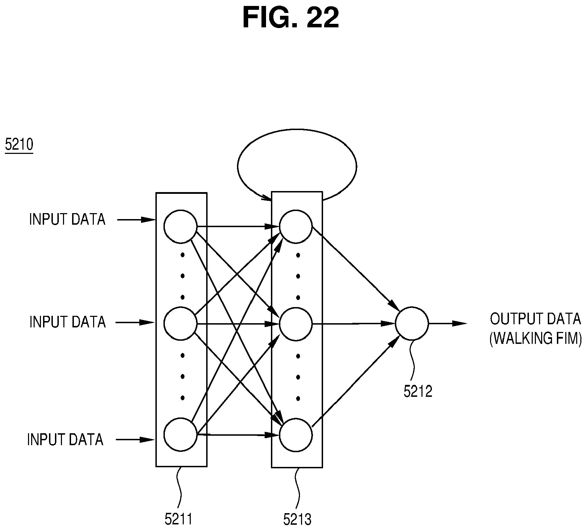

FIG. 22 illustrates a learning model 5210 according to the present embodiment. Specifically, the learning model 5210 is an RNN that includes an input layer 5211, an intermediate layer 5213, and an output layer 5212. Data sets are sequentially input to the input layer 5211 in time series. In the learning model 5210 that is the RNN, the output of the intermediate layer 5213 is input to the intermediate layer 5213 again.

As described above, the data generation unit 510a sets, as one data set, each of the determination results of abnormal walking and the data before and after the change of the setting parameter. As such, the learning unit 510b can appropriately perform machine learning. In other words, the data generation unit 510a can generate a learned model with higher accuracy by simple calculation.

### First Embodiment Example

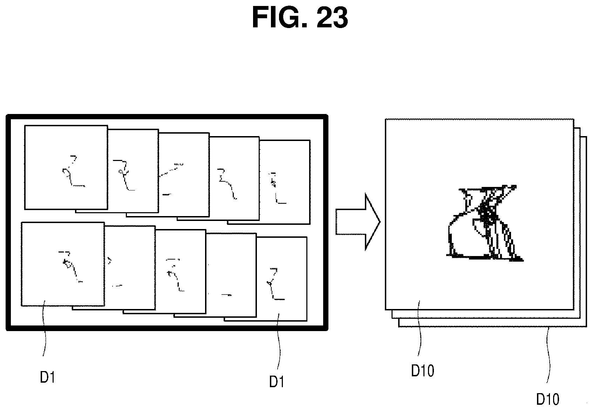

The learned model according to a first embodiment example will be described with reference to FIG. 23. FIG. 23 is a diagram for describing the learning data used to establish the learned model. In the present embodiment example, the data generation unit 510a uses two-dimensional image data as the detected data serving as the data for learning. Specifically, the load sensor 222 installed in the walking assistive device 120 detects the load on the sole. Then, the auxiliary control unit 220 obtains a center of the load (COP: Center of Pressure). Since a sampling rate of the load sensor 222 may be, for example, 30 milliseconds, the COP becomes the log data at intervals of 30 milliseconds. The data generation unit 510a cuts out the log data of the COP for one walking cycle, and uses it as tracing data D1. The tracing data D1 may be, for example, a 1-bit image of 100 pixels wide and 100 pixels high. In addition, one walking cycle is the time corresponding to one step of the injured leg, that is, the time period from when the injured leg contacts the ground to when it lifts off the ground.

Further, the data generation unit 510a integrates 10 pieces of tracing data D1 into one piece of tracing data D10. In other words, the tracing data D10 shows, as two-dimensional image data, the tracing of the COP for 10 walking cycles in one segment of walking training. The tracing data D10 may be, for example, a 1-bit image of 100 pixels wide and 100 pixels high.

The data generation unit 510a generates a plurality of pieces of tracing data D10 from the rehabilitation data collected from the plurality of trainees 900. Further, the data generation unit 510a attaches the actual measurement value of the walking FIM to each piece of tracing data D10 as the supervised data (a correct answer label). In other words, the data generation unit 510a sets the tracing data D10 and the actual measurement values of the walking FIM as one data set for learning.

In the present embodiment example, 2400 sets are prepared. Specifically, the rehabilitation data for 24,000 steps is collected from walking training of 30 trainees 900. The learning unit 510b mounts the above data sets on the CNN model. Then, a verification test is performed using the learned model established by the learning unit 510b. In other words, the server 500 collects the COP data as the rehabilitation data from walking training of the trainees 900 other than the trainee 900 from which the data for learning has been collected. Thereafter, the data generation unit 510a generates the tracing data D10 from the COP data for 10 walking cycles, and sets the tracing data D10 as input data of the learned model. The verification accuracy in the verification test was approximately 0.625.

### Second Embodiment Example

In a second embodiment example, the setting parameter is added to the data set for learning in the first embodiment example. In other words, the data generation unit 510a generates the data for learning by setting the tracing data D10, the actual measurement value of the walking FIM, and the setting parameter as one data set. As described above, examples of the setting parameter include the assisting level and the load support amount in the walking training device 100. The setting parameter may be, for example, a 10-dimensional or 16-dimensional vector. The learning unit 510b attaches the actual measurement value of the walking FIM to the tracing data D10 and the setting parameter as the supervised data (the correct answer label).

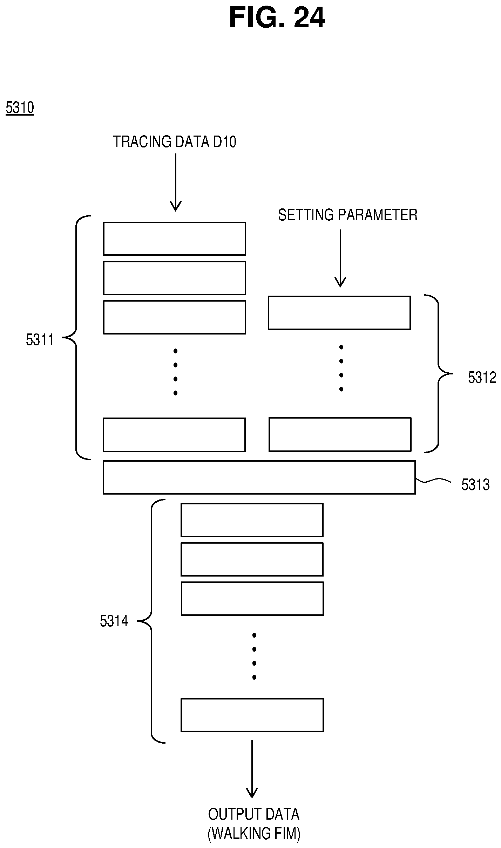

The learning unit 510b establishes the learning model 5310 illustrated in FIG. 24. The learning model 5310 includes a feature extraction unit 5311, a MLP 5312, a connection layer 5313, and a full connection layer 5314. The feature extraction unit 5311 extracts features of the tracing data D10, which is a two-dimensional image, upon receiving an input of the tracing data D10. For example, the learning unit 510b may use part or all of the learned model established in the first embodiment example as the feature extraction unit 5311.

The MLP 5312 uses the setting parameter as the input data. The connection layer 5313 connects the feature extraction unit 5311 and the MLP 5312. In other words, the outputs of the feature extraction unit 5311 and the MLP 5312 are input to the connection layer 5313. When the output of the connection layer 5313 passes through the full connection layer 5314, a predicted value of the walking FIM is output.

The verification accuracy of the second embodiment example was 0.841. Therefore, a learned model with higher accuracy could be established by using the setting parameter as the input data.

### Third Embodiment Example

In a third embodiment example, the initial walking FIM is added to the data set of the second embodiment example. Moreover, the initial walking FIM is a value actually measured before the start of rehabilitation in order to assess the severity of symptoms of a hemiplegic patient. For example, the initial walking FIM is a value measured for the trainee 900 who has suffered a stroke, or the like, before the initial walking training after a surgery. Alternatively, the initial walking FIM may be a flat ground walking FIM measured for the first time during hospitalization. The input rate of initial walking FIM is higher as the walking training is appropriately performed. Therefore, it is easy to use the initial walking FIM as the learning data. The initial walking FIM is input to the MLP 5312 together with the setting parameter.

The verification accuracy of the third embodiment example was 0.884. Therefore, a learned model with higher accuracy could be established by using the setting parameter and the initial walking FIM as the input data. The prediction accuracy could be improved by performing machine learning using the trainee data, such as the initial walking FIM.

Further, part or all of the processing in the server 500 or the walking training device 100 described above may be realized as a computer program. In addition, the learned model performed in the server 500 or the walking training device 100 may be realized as a computer program. For example, when the server 500 or the walking training device 100 performs the learned model as a computer program, a predicted value of the walking FIM, or the like is output.

Such a program may be stored and provided to a computer using various types of non-transitory computer-readable media. The non-transitory computer-readable media includes various types of tangible storage media. Examples of non-transitory computer-readable media include magnetic recording media (for example, a flexible disk, a magnetic tape, and a hard disk drive), magneto-optical recording media (for example, a magneto-optical disk), a read-only memory (CD-ROM), a CD-R, a CD-R/W, a semiconductor memory (for example, a mask ROM, a programmable ROM (PROM), an erasable PROM (EPROM), a flash ROM, and a random access memory (RAM)). Moreover, the program may be provided to the computer by various types of temporary computer-readable media. Examples of temporary computer-readable media include an electrical signal, an optical signal, and electromagnetic waves. The temporary computer-readable media can provide the program to the computer via a wired communication line, such as an electric wire and an optical fiber, or a wireless communication line.

The present invention is not limited to the above embodiments, and can be appropriately modified within the scope of the present invention.

## Claims

1. A rehabilitation support system comprising:
an actuator configured to assist a rehabilitation movement of a trainee;
a sensor configured to detect data on the rehabilitation movement assisted by the actuator; and
a storage medium configured to store a learned model that outputs an index indicating a movement ability of the trainee when the actuator is not used, upon receiving an input of detected data corresponding to a detection result by the sensor, wherein:
the rehabilitation support system is a walking training system configured to perform walking training for the trainee;
the actuator is configured to assist a walking movement of the trainee; and
the index is a predicted value of a walking ability of the trainee when the actuator is not used.

2. The rehabilitation support system according to claim 1, wherein:
the sensor is provided to detect a plurality of movement amounts in the walking movement of the trainee;
the rehabilitation support system is configured to assess that the walking movement is abnormal when at least one of the movement amounts satisfies any of predetermined abnormal walking criteria; and
the detected data includes an assessment result on whether the walking movement is abnormal.

3. The rehabilitation support system according to claims 1 or 2, wherein the learned model outputs the index upon receiving an input of a setting parameter on setting of the actuator.

4. The rehabilitation support system according to any one of claims 1 to 3, wherein the learned model outputs the index upon receiving an input of trainee data on the trainee.

5. The rehabilitation support system according to any one of claims 1 to 4, wherein:
the actuator is configured to assist rehabilitation movements of a plurality of trainees;
the trainees are classified into groups according to trainee data on the trainees; and
a different learned model is set for each of the groups.

6. A prediction device that predicts a movement ability of a trainee that performs rehabilitation with a rehabilitation support device, the prediction device comprising:
a data acquisition unit (520) configured to acquire rehabilitation data on rehabilitation of the trainee; and
a prediction unit (510) configured to predict the movement ability based on the rehabilitation data, wherein:
the rehabilitation support device is a walking training device configured to perform walking training for the trainee, the rehabilitation support device including:
an actuator configured to assist a rehabilitation movement and a walking movement of the trainee; and
a sensor configured to detect data on the rehabilitation movement assisted by the actuator;
the rehabilitation data includes detected data corresponding to a detection result by the sensor; and
the prediction unit (510) is configured to use a learned model that outputs an index indicating the movement ability when the actuator is not used upon receiving an input of the detected data, wherein
the index is a predicted value of a walking ability of the trainee when the actuator is not used.

7. A prediction method of predicting a movement ability of a trainee that performs rehabilitation with a rehabilitation support device, the prediction method comprising:
acquiring rehabilitation data on rehabilitation of the trainee; and
predicting the movement ability based on the rehabilitation data, wherein:
the rehabilitation support device is a walking training device configured to perform walking training for the trainee, the rehabilitation support device including:
an actuator configured to assist a rehabilitation movement and a walking movement of the trainee; and
a sensor configured to detect data on the rehabilitation movement assisted by the actuator; and
the rehabilitation data includes detected data corresponding to a detection result from the sensor, and
a learned model is used in predicting the movement ability, the learned model outputting an index indicating the movement ability when the actuator is not used, upon receiving an input of the detected data, wherein
the index is a predicted value of a walking ability of the trainee when the actuator is not used.

8. A non-transitory storage medium storing instructions that are executable by one or more processors and that cause the one or more processors to execute the prediction method according to claim 7.

9. A learning device comprising:
A data acquisition unit (520) configured to acquire rehabilitation data from a rehabilitation support device including an actuator configured to assist a rehabilitation movement of a trainee and a sensor configured to detect data on the rehabilitation movement assisted by the actuator, wherein
the rehabilitation support device is a walking training device configured to perform walking training for the trainee, and
the actuator is configured to assist a walking movement of the trainee;
a data generation unit (510a) configured to generate, as data for learning, an index indicating a movement ability of the trainee and the rehabilitation data that includes detected data corresponding to a detection result by the sensor; and
a learning unit (510b) configured to generate, by performing machine learning using the data for learning, a learning model that outputs an index indicating the movement ability of the trainee when the actuator is not used, upon receiving an input of the detected data, wherein
the index output by the learning model is a predicted value of a walking ability of the trainee when the actuator is not used.

10. The learning device according to claim 9, wherein:
the rehabilitation data includes, as an index indicating the movement ability of the trainee, an actual measurement value of a walking ability of the trainee when the actuator is not used; and
the learning unit (510b) is configured to perform learning, using the actual measurement value of the walking ability as supervised data.

11. The learning device according to claim 10, wherein:
the sensor is provided to detect a plurality of movement amounts in the walking movement of the trainee;
the rehabilitation support device is configured to assess that the walking movement is abnormal when at least one of the movement amounts satisfies any of predetermined abnormal walking criteria; and
the rehabilitation data includes an assessment result on whether the walking movement is abnormal as the detected data.

12. The learning device according to any one of claims 9 to 11, wherein:
the data acquisition unit (520) is configured to acquire a setting parameter on setting of the actuator as the rehabilitation data; and
the learning unit (5 10b) is configured to generate a learned model that outputs the index upon receiving an input of the setting parameter.

13. The learning device according to any one of claims 9 to 12, wherein:
the learning unit (510b) is configured to generate a learning model that outputs the index upon receiving an input of trainee data on the trainee.

14. The learning device according to any one of claims 9 to 13, wherein:
the actuator is configured to assist rehabilitation movements of a plurality of trainees;
the trainees are classified into groups according to trainee data on the trainees; and
the learning unit (5 10b) is configured to generate a different learning model for each of the groups.

15. A non-transitory storage medium storing a learned model that is executable by one or more processors and that causes the one or more processors to function to predict a movement ability of a trainee based on rehabilitation data for assessment acquired by a rehabilitation support device, wherein the learned model is the learning model generated by the learning device according to any one of claims 9 to 14.

16. The non-transitory storage medium according to claim 15, wherein the detected data and the index are learned in association with each other in the learned model.

17. A learning method comprising:
acquiring rehabilitation data from a rehabilitation support device including an actuator configured to assist a rehabilitation movement of a trainee and a sensor configured to detect data on the rehabilitation movement assisted by the actuator, wherein
the rehabilitation support device is a walking training device configured to perform walking training for the trainee, and
the actuator is configured to assist a walking movement of the trainee;
generating, as data for learning, an index indicating a movement ability of the trainee and the rehabilitation data that includes detected data corresponding to a detection result by the sensor; and
generating, by performing machine learning using the data for learning, a learning model that outputs an index indicating the movement ability of the trainee when the actuator is not used, upon receiving an input of the detected data, wherein
the index output by the learning model is a predicted value of a walking ability of the trainee when the actuator is not used.

18. A non-transitory storage medium storing instructions that are executable by one or more processors and that cause the one or more processors to execute the learning method according to claim 17.

19. An operation method of operating a rehabilitation support system including an actuator configured to assist a rehabilitation movement of a trainee and a sensor configured to detect data on the rehabilitation movement assisted by the actuator, wherein the rehabilitation support device is a walking training device configured to perform walking training for the trainee and the actuator is configured to assist a walking movement of the trainee, the operation method comprising:
acquiring detected data corresponding to a detection result by the sensor; and
outputting by using a learned model an index indicating a movement ability of the trainee when the actuator is not used, upon receiving an input of the detected data, wherein
the index is a predicted value of a walking ability of the trainee when the actuator is not used.

## Patentansprüche

1. Rehabilitationsunterstützungssystem, umfassend:
einen Aktuator, der dazu ausgelegt ist, eine Rehabilitationsbewegung eines Trainierenden zu unterstützen;
einen Sensor, der dazu ausgelegt ist, Daten zu der durch den Aktuator unterstützten Rehabilitationsbewegung zu detektieren; und
ein Speichermedium, das dazu ausgelegt ist, ein gelerntes Modell zu speichern, das einen Index, der eine Bewegungsfähigkeit des Trainierenden, wenn der Aktuator nicht verwendet wird, anzeigt, bei Empfangen einer Eingabe von detektierten Daten ausgibt, die einem Detektionsergebnis durch den Sensor entsprechen, wobei:
das Rehabilitationsunterstützungssystem ein Gehtrainingssystem ist, das dazu ausgelegt ist, ein Gehtraining für den Trainierenden durchzuführen;
der Aktuator dazu ausgelegt ist, eine Gehbewegung des Trainierenden zu unterstützen; und
der Index ein vorhergesagter Wert einer Gehfähigkeit des Trainierenden, wenn der Aktuator nicht verwendet wird, ist.

2. Rehabilitationsunterstützungssystem nach Anspruch 1, wobei:
der Sensor bereitgestellt ist, um eine Mehrzahl von Bewegungsausmaßen bei der Gehbewegung des Trainierenden zu detektieren;
das Rehabilitationsunterstützungssystem dazu ausgelegt ist, zu beurteilen, dass die Gehbewegung abnormal ist, wenn mindestens eines der Bewegungsausmaße ein beliebiges von vorbestimmten Kriterien für ein abnormales Gehen erfüllt; und
die detektierten Daten ein Beurteilungsergebnis dazu umfassen, ob die Gehbewegung abnormal ist.

3. Rehabilitationsunterstützungssystem nach Anspruch 1 oder 2, wobei das gelernte Modell den Index bei Empfangen einer Eingabe eines Einstellungsparameters zu einer Einstellung des Aktuators ausgibt.

4. Rehabilitationsunterstützungssystem nach einem der Ansprüche 1 bis 3, wobei das gelernte Modell den Index bei Empfangen einer Eingabe von Trainierendendaten zu dem Trainierenden ausgibt.

5. Rehabilitationsunterstützungssystem nach einem der Ansprüche 1 bis 4, wobei:
der Aktuator dazu ausgelegt ist, Rehabilitationsbewegungen einer Mehrzahl von Trainierenden zu unterstützen; wobei die Trainierenden gemäß Trainierendendaten zu den Trainierenden in Gruppen eingestuft sind; und wobei ein unterschiedliches gelerntes Modell für jede der Gruppen eingestellt ist.

6. Vorhersagevorrichtung, die eine Bewegungsfähigkeit eines Trainierenden vorhersagt, der eine Rehabilitation mit einer Rehabilitationsunterstützungsvorrichtung durchführt, wobei die Vorhersagevorrichtung umfasst:
eine Datenerfassungseinheit (520), die dazu ausgelegt ist, Rehabilitationsdaten zu einer Rehabilitation des Trainierenden zu erfassen; und
eine Vorhersageeinheit (510), die dazu ausgelegt ist, die Bewegungsfähigkeit basierend auf den Rehabilitationsdaten vorherzusagen, wobei:
die Rehabilitationsunterstützungsvorrichtung eine Gehtrainingsvorrichtung ist, die dazu ausgelegt ist, ein Gehtraining für den Trainierenden durchzuführen, wobei die Rehabilitationsunterstützungsvorrichtung umfasst:
einen Aktuator, der dazu ausgelegt ist, eine Rehabilitationsbewegung und eine Gehbewegung des Trainierenden zu unterstützen; und
einen Sensor, der dazu ausgelegt ist, Daten zu der durch den Aktuator unterstützten Rehabilitationsbewegung zu detektieren;
wobei die Rehabilitationsdaten detektierte Daten umfassen, die einem Detektionsergebnis durch den Sensor entsprechen; und
wobei die Vorhersageeinheit (510) dazu ausgelegt ist, ein gelerntes Modell zu verwenden, das einen Index, der die Bewegungsfähigkeit, wenn der Aktuator nicht verwendet wird, anzeigt, bei Empfangen einer Eingabe der detektierten Daten ausgibt, wobei
der Index ein vorhergesagter Wert einer Gehfähigkeit des Trainierenden, wenn der Aktuator nicht verwendet wird, ist.

7. Vorhersageverfahren zum Vorhersagen einer Bewegungsfähigkeit eines Trainierenden, der eine Rehabilitation mit einer Rehabilitationsunterstützungsvorrichtung durchführt, wobei das Vorhersageverfahren umfasst:
Erfassen von Rehabilitationsdaten zu einer Rehabilitation des Trainierenden; und
Vorhersagen der Bewegungsfähigkeit basierend auf den Rehabilitationsdaten, wobei:
die Rehabilitationsunterstützungsvorrichtung eine Gehtrainingsvorrichtung ist, die dazu ausgelegt ist, ein Gehtraining für den Trainierenden durchzuführen, wobei die Rehabilitationsunterstützungsvorrichtung umfasst:
einen Aktuator, der dazu ausgelegt ist, eine Rehabilitationsbewegung und eine Gehbewegung des Trainierenden zu unterstützen; und
einen Sensor, der dazu ausgelegt ist, Daten zu der durch den Aktuator unterstützten Rehabilitationsbewegung zu detektieren; und
wobei die Rehabilitationsdaten detektierte Daten umfassen, die einem Detektionsergebnis von dem Sensor entsprechen, und
wobei ein gelerntes Modell bei Vorhersagen der Bewegungsfähigkeit verwendet wird, wobei das gelernte Modell einen Index, der die Bewegungsfähigkeit, wenn der Aktuator nicht verwendet wird, anzeigt, bei Empfangen einer Eingabe der detektierten Daten ausgibt, wobei
der Index ein vorhergesagter Wert einer Gehfähigkeit des Trainierenden, wenn der Aktuator nicht verwendet wird, ist.

8. Nichtflüchtiges Speichermedium, das Anweisungen speichert, die durch einen oder mehrere Prozessoren ausführbar sind und die den einen oder die mehreren Prozessoren veranlassen, das Vorhersageverfahren nach Anspruch 7 auszuführen.

9. Lernvorrichtung, umfassend:
eine Datenerfassungseinheit (520), die dazu ausgelegt ist, Rehabilitationsdaten von einer Rehabilitationsunterstützungsvorrichtung zu erfassen, die einen Aktuator, der dazu ausgelegt ist, eine Rehabilitationsbewegung eines Trainierenden zu unterstützen, und einen Sensor, der dazu ausgelegt ist, Daten zu der durch den Aktuator unterstützten Rehabilitationsbewegung zu detektieren, umfasst, wobei
die Rehabilitationsunterstützungsvorrichtung eine Gehtrainingsvorrichtung ist, die dazu ausgelegt ist, ein Gehtraining für den Trainierenden durchzuführen, und
der Aktuator dazu ausgelegt ist, eine Gehbewegung des Trainierenden zu unterstützen;
eine Datenerzeugungseinheit (510a), die dazu ausgelegt ist, als Daten für ein Lernen einen Index, der eine Bewegungsfähigkeit des Trainierenden anzeigt, und die Rehabilitationsdaten, die einem Detektionsergebnis durch den Sensor entsprechende detektierte Daten umfassen, zu erzeugen; und
eine Lerneinheit (510b), die dazu ausgelegt ist, durch Durchführen von maschinellem Lernen unter Verwendung der Daten für ein Lernen ein Lernmodell zu erzeugen, das einen Index, der die Bewegungsfähigkeit des Trainierenden, wenn der Aktuator nicht verwendet wird, anzeigt, bei Empfangen einer Eingabe der detektierten Daten ausgibt, wobei
der durch das Lernmodell ausgegebene Index ein vorhergesagter Wert einer Gehfähigkeit des Trainierenden, wenn der Aktuator nicht verwendet wird, ist.

10. Lernvorrichtung nach Anspruch 9, wobei:
die Rehabilitationsdaten als einen Index, der die Bewegungsfähigkeit des Trainierenden anzeigt, einen tatsächlichen Messwert einer Gehfähigkeit des Trainierenden, wenn der Aktuator nicht verwendet wird, umfassen; und
die Lerneinheit (510b) dazu ausgelegt ist, ein Lernen unter Verwendung des tatsächlichen Messwerts der Gehfähigkeit als überwachte Daten durchzuführen.

11. Lernvorrichtung nach Anspruch 10, wobei:
der Sensor bereitgestellt ist, um eine Mehrzahl von Bewegungsausmaßen bei der Gehbewegung des Trainierenden zu detektieren;
die Rehabilitationsunterstützungsvorrichtung dazu ausgelegt ist, zu beurteilen, dass die Gehbewegung abnormal ist, wenn mindestens eines der Bewegungsausmaße ein beliebiges von vorbestimmten Kriterien für ein abnormales Gehen erfüllt; und
die Rehabilitationsdaten ein Beurteilungsergebnis dazu, ob die Gehbewegung abnormal ist, als die detektierten Daten umfassen.

12. Lernvorrichtung nach einem der Ansprüche 9 bis 11, wobei:
die Datenerfassungseinheit (520) dazu ausgelegt ist, einen Einstellungsparameter zu einer Einstellung des Aktuators als die Rehabilitationsdaten zu erfassen; und
die Lerneinheit (510b) dazu ausgelegt ist, ein gelerntes Modell zu erzeugen, das den Index bei Empfangen einer Eingabe des Einstellungsparameters ausgibt.

13. Lernvorrichtung nach einem der Ansprüche 9 bis 12, wobei:
die Lerneinheit (510b) dazu ausgelegt ist, ein Lernmodell zu erzeugen, das den Index bei Empfangen einer Eingabe von Trainierendendaten zu dem Trainierenden ausgibt.

14. Lernvorrichtung nach einem der Ansprüche 9 bis 13, wobei:
der Aktuator dazu ausgelegt ist, Rehabilitationsbewegungen einer Mehrzahl von Trainierenden zu unterstützen;
die Trainierenden gemäß Trainierendendaten zu den Trainierenden in Gruppen eingestuft sind; und
die Lerneinheit (510b) dazu ausgelegt ist, für jede der Gruppen ein unterschiedliches Lernmodell zu erzeugen.

15. Nichtflüchtiges Speichermedium, das ein gelerntes Modell speichert, das durch einen oder mehrere Prozessoren ausführbar ist und das den einen oder die mehreren Prozessoren veranlasst, dazu zu dienen, eine Bewegungsfähigkeit eines Trainierenden basierend auf durch eine Rehabilitationsunterstützungsvorrichtung erfassten Rehabilitationsdaten für eine Beurteilung vorherzusagen, wobei das gelernte Modell das durch die Lernvorrichtung nach einem der Ansprüche 9 bis 14 erzeugte Lernmodell ist.

16. Nichtflüchtiges Speichermedium nach Anspruch 15, wobei die detektierten Daten und der Index in Verbindung miteinander in dem gelernten Modell gelernt werden.

17. Lernverfahren, umfassend:
Erfassen von Rehabilitationsdaten von einer Rehabilitationsunterstützungsvorrichtung, die einen Aktuator, der dazu ausgelegt ist, eine Rehabilitationsbewegung eines Trainierenden zu unterstützen, und einen Sensor, der dazu ausgelegt ist, Daten zu der durch den Aktuator unterstützten Rehabilitationsbewegung zu detektieren, umfasst, wobei
die Rehabilitationsunterstützungsvorrichtung eine Gehtrainingsvorrichtung ist, die dazu ausgelegt ist, ein Gehtraining für den Trainierenden durchzuführen, und
der Aktuator dazu ausgelegt ist, eine Gehbewegung des Trainierenden zu unterstützen;
Erzeugen, als Daten für ein Lernen, eines Index, der eine Bewegungsfähigkeit des Trainierenden anzeigt, und der Rehabilitationsdaten, die einem Detektionsergebnis durch den Sensor entsprechende detektierte Daten umfassen; und
Erzeugen, durch Durchführen von maschinellem Lernen unter Verwendung der Daten für ein Lernen, eines Lernmodells, das einen Index, der die Bewegungsfähigkeit des Trainierenden, wenn der Aktuator nicht verwendet wird, anzeigt, bei Empfangen einer Eingabe der detektierten Daten ausgibt, wobei
der durch das Lernmodell ausgegebene Index ein vorhergesagter Wert einer Gehfähigkeit des Trainierenden, wenn der Aktuator nicht verwendet wird, ist.

18. Nichtflüchtiges Speichermedium, das Anweisungen speichert, die durch einen oder mehrere Prozessoren ausführbar sind und die den einen oder die mehreren Prozessoren veranlassen, das Lernverfahren nach Anspruch 17 auszuführen.

19. Betriebsverfahren zum Betreiben eines Rehabilitationsunterstützungssystems, das einen Aktuator, der dazu ausgelegt ist, eine Rehabilitationsbewegung eines Trainierenden zu unterstützen, und einen Sensor, der dazu ausgelegt ist, Daten zu der durch den Aktuator unterstützten Rehabilitationsbewegung zu detektieren, umfasst, wobei die Rehabilitationsunterstützungsvorrichtung eine Gehtrainingsvorrichtung ist, die dazu ausgelegt ist, ein Gehtraining für den Trainierenden durchzuführen, und der Aktuator dazu ausgelegt ist, eine Gehbewegung des Trainierenden zu unterstützen, wobei das Betriebsverfahren umfasst:
Erfassen von detektierten Daten, die einem Detektionsergebnis durch den Sensor entsprechen; und
Ausgeben, unter Verwendung eines gelernten Modells, eines Index, der eine Bewegungsfähigkeit des Trainierenden, wenn der Aktuator nicht verwendet wird, anzeigt, bei Empfangen einer Eingabe der detektierten Daten, wobei
der Index ein vorhergesagter Wert einer Gehfähigkeit des Trainierenden, wenn der Aktuator nicht verwendet wird, ist.

## Revendications

1. Système d'aide à la rééducation, comprenant :
un actionneur configuré pour assister un mouvement de rééducation d'un patient ;
un capteur configuré pour détecter des données sur le mouvement de rééducation assisté par l'actionneur ; et
un support de stockage configuré pour stocker un modèle appris qui émet un index indiquant une capacité de mouvement du patient lorsque l'actionneur n'est pas utilisé, après réception d'une entrée de données détectées correspondant à un résultat de détection par le capteur,
le système d'aide à la rééducation étant un système d'entraînement à la marche configuré pour réaliser un entraînement à la marche pour le patient ;
l'actionneur étant configuré pour assister un mouvement de marche du patient ; et
l'index étant une valeur prédite d'une capacité de marche du patient lorsque l'actionneur n'est pas utilisé.

2. Système d'aide à la rééducation selon la revendication 1,
le capteur étant fourni pour détecter une pluralité de quantités de mouvement dans le mouvement de marche du patient ;
le système d'aide à la rééducation étant configuré pour évaluer que le mouvement de marche est anormal lorsqu'au moins l'une des quantités de mouvement satisfait l'un quelconque de critères de marche anormale prédéterminés ; et
les données détectées incluant un résultat d'évaluation indiquant si le mouvement de marche est anormal.

3. Système d'aide à la rééducation selon les revendications 1 ou 2, dans lequel le modèle appris émet l'index après réception d'une entrée d'un paramètre de réglage sur le réglage de l'actionneur.

4. Système d'aide à la rééducation selon l'une quelconque des revendications 1 à 3, dans lequel le modèle appris émet l'index après réception d'une entrée de données de patient sur le patient.

5. Système d'aide à la rééducation selon l'une quelconque des revendications 1 à 4, dans lequel :
l'actionneur est configuré pour assister des mouvements de rééducation d'une pluralité de patients ; les patients sont classifiés en groupes en fonction de données de patient sur les patients ; et un modèle appris différent est défini pour chacun des groupes.

6. Dispositif de prédiction qui prédit une capacité de mouvement d'un patient qui réalise une rééducation avec un dispositif d'aide à la rééducation, le dispositif de prédiction comprenant :
une unité d'acquisition de données (520) configurée pour acquérir des données de rééducation sur la rééducation du patient ; et
une unité de prédiction (510) configurée pour prédire la capacité de mouvement sur la base des données de rééducation, dans lequel :
le dispositif d'aide à la rééducation est un dispositif d'entraînement à la marche configuré pour réaliser un entraînement à la marche pour le patient, le dispositif d'aide à la rééducation incluant :
un actionneur configuré pour assister un mouvement de rééducation et un mouvement de marche du patient ; et
un capteur configuré pour détecter des données sur le mouvement de rééducation assisté par l'actionneur ;
les données de rééducation incluent des données détectées correspondant à un résultat de détection par le capteur ; et
l'unité de prédiction (510) est configurée pour utiliser un modèle appris qui émet un index indiquant la capacité de mouvement lorsque l'actionneur n'est pas utilisé après réception d'une entrée des données détectées, dans lequel l'index est une valeur prédite d'une capacité de marche du patient lorsque l'actionneur n'est pas utilisé.

7. Procédé de prédiction prédisant une capacité de mouvement d'un patient qui réalise une rééducation avec un dispositif d'aide à la rééducation, le procédé de prédiction comprenant : l'acquisition de données de rééducation sur la rééducation du patient ; et
la prédiction de la capacité de mouvement sur la base des données de rééducation, dans lequel :
le dispositif d'aide à la rééducation est un dispositif d'entraînement à la marche configuré pour réaliser un entraînement à la marche pour le patient, le dispositif d'aide à la rééducation incluant :
un actionneur configuré pour assister un mouvement de rééducation et un mouvement de marche du patient ; et un capteur configuré pour détecter des données sur le mouvement de rééducation assisté par l'actionneur ; et les données de rééducation incluent des données détectées correspondant à un résultat de détection provenant du capteur, et
un modèle appris est utilisé dans la prédiction de la capacité de mouvement, le modèle appris émettant un index indiquant la capacité de mouvement lorsque l'actionneur n'est pas utilisé, après réception d'une entrée des données détectées, dans lequel
l'index est une valeur prédite d'une capacité de marche du patient lorsque l'actionneur n'est pas utilisé.

8. Support de stockage non transitoire stockant des instructions qui peuvent être exécutées par un ou plusieurs processeurs et qui amènent les un ou plusieurs processeurs à exécuter le procédé de prédiction selon la revendication 7.

9. Dispositif d'apprentissage comprenant :
une unité d'acquisition de données (520) configurée pour acquérir des données de rééducation provenant d'un dispositif d'aide à la rééducation incluant un actionneur configuré pour assister un mouvement de rééducation d'un patient et un capteur configuré pour détecter des données sur le mouvement de rééducation assisté par l'actionneur, dans lequel
le dispositif d'aide à la rééducation est un dispositif d'entraînement à la marche configuré pour réaliser un entraînement à la marche pour le patient, et
l'actionneur est configuré pour assister un mouvement de marche du patient ;
une unité de génération de données (510a) configurée pour générer, comme données d'apprentissage, un index indiquant une capacité de mouvement du patient et les données de rééducation qui incluent des données détectées correspondant à un résultat de détection par le capteur ; et
une unité d'apprentissage (510b) configurée pour générer, en réalisant un apprentissage automatique à l'aide des données d'apprentissage, un modèle d'apprentissage qui émet un index indiquant la capacité de mouvement du patient lorsque l'actionneur n'est pas utilisé, après réception d'une entrée des données détectées, dans lequel
l'index émis par le modèle d'apprentissage est une valeur prédite d'une capacité de marche du patient lorsque l'actionneur n'est pas utilisé.

10. Dispositif d'apprentissage selon la revendication 9, dans lequel :
les données de rééducation incluent, comme index indiquant la capacité de mouvement du patient, une valeur de mesure réelle d'une capacité de marche du patient lorsque l'actionneur n'est pas utilisé ; et
l'unité d'apprentissage (510b) est configurée pour réaliser un apprentissage, à l'aide de la valeur de mesure réelle de la capacité de marche comme données supervisées.

11. Dispositif d'apprentissage selon la revendication 10, dans lequel :
le capteur est fourni pour détecter une pluralité de quantités de mouvement dans le mouvement de marche du patient ;
le dispositif d'aide à la rééducation est configuré pour évaluer que le mouvement de marche est anormal lorsqu'au moins l'une des quantités de mouvement satisfait l'un quelconque de critères de marche anormale prédéterminés ; et
les données de rééducation incluent un résultat d'évaluation indiquant si le mouvement de marche est anormal comme données détectées.

12. Dispositif d'apprentissage selon l'une quelconque des revendications 9 à 11, dans lequel :
l'unité d'acquisition de données (520) est configurée pour acquérir un paramètre de réglage sur le réglage de l'actionneur comme données de rééducation ; et
l'unité d'apprentissage (510b) est configurée pour générer un modèle appris qui émet l'index après réception d'une entrée du paramètre de réglage.

13. Dispositif d'apprentissage selon l'une quelconque des revendications 9 à 12, dans lequel :
l'unité d'apprentissage (510b) est configurée pour générer un modèle d'apprentissage qui émet l'index après réception d'une entrée de données de patient sur le patient.

14. Dispositif d'apprentissage selon l'une quelconque des revendications 9 à 13, dans lequel :
l'actionneur est configuré pour assister des mouvements de rééducation d'une pluralité de patients ;
les patients sont classifiés en groupes en fonction de données de patient sur les patients ; et
l'unité d'apprentissage (510b) est configurée pour générer un modèle d'apprentissage différent pour chacun des groupes.

15. Support de stockage non transitoire stockant un modèle appris qui peut être exécuté par un ou plusieurs processeurs et qui amène les un ou plusieurs processeurs à fonctionner pour prédire une capacité de mouvement d'un patient sur la base de données de rééducation pour une évaluation acquises par un dispositif d'aide à la rééducation, dans lequel le modèle appris est le modèle d'apprentissage généré par le dispositif d'apprentissage selon l'une quelconque des revendications 9 à 14.

16. Support de stockage non transitoire selon la revendication 15, dans lequel les données détectées et l'index sont appris en association conjointement dans le modèle appris.

17. Procédé d'apprentissage comprenant :
l'acquisition de données de rééducation provenant d'un dispositif d'aide à la rééducation incluant un actionneur configuré pour assister un mouvement de rééducation d'un patient et un capteur configuré pour détecter des données sur le mouvement de rééducation assisté par l'actionneur, dans lequel
le dispositif d'aide à la rééducation est un dispositif d'entraînement à la marche configuré pour réaliser un entraînement à la marche pour le patient, et
l'actionneur est configuré pour assister un mouvement de marche du patient ;
la génération, comme données d'apprentissage, d'un index indiquant une capacité de mouvement du patient et des données de rééducation qui incluent des données détectées correspondant à un résultat de détection par le capteur ; et
la génération, en réalisant un apprentissage automatique à l'aide des données d'apprentissage, d'un modèle d'apprentissage qui émet un index indiquant la capacité de mouvement du patient lorsque l'actionneur n'est pas utilisé, après réception d'une entrée des données détectées, dans lequel
l'index émis par le modèle d'apprentissage est une valeur prédite d'une capacité de marche du patient lorsque l'actionneur n'est pas utilisé.

18. Support de stockage non transitoire stockant des instructions qui peuvent être exécutées par un ou plusieurs processeurs et qui amènent les un ou plusieurs processeurs à exécuter le procédé d'apprentissage selon la revendication 17.

19. Procédé de fonctionnement pour faire fonctionner un système d'aide à la rééducation incluant un actionneur configuré pour assister un mouvement de rééducation d'un patient et un capteur configuré pour détecter des données sur le mouvement de rééducation assisté par l'actionneur, dans lequel le dispositif d'aide à la rééducation est un dispositif d'entraînement à la marche configuré pour réaliser un entraînement à la marche pour le patient et l'actionneur est configuré pour assister un mouvement de marche du patient, le procédé de fonctionnement comprenant :
l'acquisition de données détectées correspondant à un résultat de détection par le capteur ; et
l'émission à l'aide d'un modèle appris d'un index indiquant une capacité de mouvement du patient lorsque l'actionneur n'est pas utilisé, après réception d'une entrée des données détectées, dans lequel
l'index est une valeur prédite d'une capacité de marche du patient lorsque l'actionneur n'est pas utilisé.
